# EUROPEAN PATENT SPECIFICATION

(11) **EP 2 713 999 B1**
(45) Date of publication and mention of the grant of the patent: **01.03.2017**
(21) Application number: 12725361.5
(22) Date of filing: 28.05.2012
(51) Int. Cl.: A61K 8/58, A61K 8/37, A61K 8/898, A61Q 5/12, A61Q 5/06

(54) **COMPOSITION COMPRISING AN ALKOXYSILANE, A FATTY ESTER AND A SILICONE, AND COSMETIC USE THEREOF**
ZUBEREITUNG ENTHALTEND EIN ALKOXYSILAN, EIN FETTSÄURE ESTER UND EINE SILIKON AND DEREN KOSMETISCHE VERWENDUNG
COMPOSITION COMPRENANT UN ALCOXYSILANE, UN ESTER GRAS ET UNE SILICONE, ET SON UTILISATION EN COSMÉTIQUE

(30) Priority: 27.05.2011 FR 1154647; 29.08.2011 US 201161528637 P
(43) Date of publication of application: 09.04.2014
(73) Proprietor: L'OREAL, 75008 Paris (FR)
(72) Inventor: BOURDIN, Claire, F-92300 Levallois Perret (FR); MINOU, Patrick, F-95110 Sannois (FR)
(74) Representative: Dodin, Catherine
(86) International application number: PCT/EP2012/059926
(87) International publication number: WO 2012/163868

(56) References cited:
- EP-A1- 1 795 180
- EP-A2- 0 159 628
- EP-A2- 1 767 189
- FR-A1- 2 783 164
- FR-A1- 2 926 984
- US-A- 2 782 790
- US-A1- 2006 110 351

## Description

The present invention relates to a composition, especially a cosmetic composition, comprising at least one alkoxysilane, at least one fatty ester and at least one silicone.

Keratin fibres may suffer attack of diverse origins, for instance mechanical attack (disentangling or brushing), or chemical attack (dyeing or permanent waving). This attack has an impact on the qualities of the fibre and will lead to difficult disentangling at the time of washing the hair, and to a non-smooth, dry and uneven surface when the hair is dry. The hair is difficult to style and lacks softness.

The conditioning compositions that are currently proposed comprise essentially cationic surfactants, fatty substances, silicones and cationic polymers. They make it possible to facilitate disentangling by softening the keratin fibre and provide sheen, softness, and uniformity to dried hair. However, these effects do not withstand shampooing, and the application of these compositions is essential at each washing step in order to treat and facilitate the disentangling of the hair.

Moreover, it has been found that consumers are increasingly in search of care compositions that are not only capable of appropriately conditioning the hair, but also capable of affording satisfactory styling effects.

In particular, people with fine or curly hair are generally in search of care products that afford styling effects that give body and volume to fine hair and curl definition to curly hair.

It has already been proposed especially in FR 2 783 164 to use alkoxysilanes to give the hair styling properties. However, aminosilane-based compositions have problems of stability on storage and also incompatibilities with components widely used in cosmetics such as certain classes of surfactants and of conditioning agents.

Furthermore, the results obtained are not completely satisfactory either as regards the cosmetic properties themselves or their durability.

There thus exists a need to have available more effective conditioning and styling compositions.

The Applicant Company has discovered, surprisingly, that compositions comprising at least one alkoxysilane, at least one fatty ester and at least one silicone make it possible to solve the problems raised above.

More specifically, one subject of the invention is a composition, especially a cosmetic composition, comprising:
(i) one or more alkoxysilanes,
(ii) one or more fatty esters; and
(iii) one or more silicones.

Such a composition is easy to distribute over the hair, easy to remove with water and leads to shiny, smooth, soft hair that is easy to disentangle. The composition affords densification to the head of hair (gives the impression of a larger number of hairs), body, volume and ease of shaping, in particular for fine hair. Finally, the compositions according to the invention also make it possible to give curly hair styling effects, especially in terms of curl definition and control.

The styling and cosmetic properties last over time, even after shampooing several times.

A further subject of the invention is a method for the nontherapeutic cosmetic treatment of keratin materials, in particular the hair, comprising the application, to said materials, of a composition as described above.

A subject of the invention is also the use of a composition according to the invention for caring for and shaping keratin materials, especially keratin fibres and in particular human keratin fibres such as the hair.

The composition according to the invention comprises one or more alkoxysilanes.

The alkoxysilanes present in the composition according to the invention are preferably chosen from organosilanes comprising one, two or three silicon atoms, preferably one or two silicon atoms.

The alkoxysilanes present in the composition according to the invention may comprise two or more hydrolysable or hydroxyl groups per molecule. The hydrolysable groups are preferably alkoxy, aryloxy or halogen groups. They may optionally comprise other chemical functions, such as salified or non-salified amine, salified or non-salified carboxylic acid, salified or non-salified sulfonic acid, salified or non-salified phosphoric acid, salified or non-salified sulfuric acid, and aldehyde, polyalcohol or polyether functions.

Preferably, the alkoxysilanes of the invention comprise one or more amine or aldehyde functions.

Even more preferably, the alkoxysilanes of the invention comprise one or more amine functions.

When the alkoxysilane present in the composition according to the invention comprises one or more amine functions, they are preferably primary amines (-NH₂) and/or secondary amines (-NH_{R}).

According to one particular embodiment, the alkoxysilane(s) present in the composition according to the invention are chosen from the compounds of formula (I): 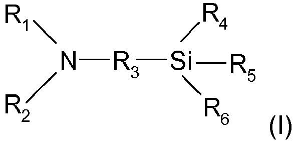 in which:
R₄ represents a halogen or a group ORₐ or R₁ₐ;
R₅ represents a halogen or a group OR_{b} or R₂ₐ;
R₆ represents a halogen or a group OR_{c} or R₃ₐ;
R₁ and R₂, represent, independently of each other, a hydrogen atom, a saturated or unsaturated, linear or branched hydrocarbon-based group, optionally substituted with an amine function, which itself may bear a substitution with a saturated or unsaturated, linear or branched hydrocarbon-based group, possibly bearing an amine function, preferably R₁ or R₂ necessarily denoting a hydrogen atom,
R₃, Rₐ, R_{b}, R_{c}, R₁ₐ, R₂ₐ and R₃ₐ represent, independently of each other, a saturated or unsaturated, linear or branched hydrocarbon-based group, optionally bearing additional chemical groups such as acid or amine groups, Rₐ, R_{b} and R_{c} also possibly denoting hydrogen, and at least two of the groups R₄, R₅ and R₆ being other than groups R₁ₐ, R₂ₐ and R₃ₐ, and preferably at least two of the groups Rₐ, R_{b} and R_{c} being other than hydrogen.

Preferably, the groups R₁, R₂, Rₐ, R₁ₐ, R₂ₐ, R₃ₐ, R_{b} and R_{c} are chosen from C₁-C₁₂ alkyl, C₅-C₁₄ aryl, (C₁-C₈)alkyl(C₅-C₁₄)aryl and (C₅-C₁₄)aryl(C₁-C₈)alkyl radicals.

Preferably, the group R₃ is chosen from C₁-C₁₂ alkylene radicals, optionally substituted with an amino, C₅-C₁₄ arylene, (C₁-C₈)alkylene(C₅-C₁₄)arylene or (C₅-C₁₄)arylene(C₁-C₈)alkylene group.

According to one particular embodiment, the alkoxysilane(s) corresponding to formula (I) are preferably 3-aminopropyltriethoxysilane, 3-aminopropylmethyldiethoxysilane, N-(2-aminoethyl)-3-aminopropylltriethoxysilane and 3-(2-aminoethylamino)propylmethyldiethoxysilane.

According to another particular embodiment, the alkoxysilane(s) used according to the invention are chosen from the compounds of formula (II):

(R₂₁O)x(R₂₂)_{y}Si-(B)p-[NR₂₃-(Bₐ)pa]q-[NR₂₃ₐ-(B_{b})pb]qa-Si(R₂₂ₐ)ya(OR₂₁ₐ)xa

in which: R₂₁, R₂₂, R₂₁ₐ and R₂₂ₐ represent, independently of each other, a saturated or unsaturated, linear or branched hydrocarbon-based chain, optionally containing one or more heteroatoms, optionally interrupted or substituted with one or more groups chosen from ether, ester, amine, amide, carboxyl, hydroxyl and carbonyl groups,
x is an integer ranging from 1 to 3, y = 3-x, xa is an integer ranging from 1 to 3, ya = 3-xa, p = 0 or 1, pa = 0 or 1, pb = 0 or 1, q = 0 or 1, qa = 0 or 1, it being understood that at least q or qa is other than zero,
B, Bₐ and B_{b} each independently represent a linear or branched divalent C₁-C₂₀ alkylene radical.
R₂₃ and R₂₃ₐ each independently represent a hydrogen atom or a saturated or unsaturated, linear or branched hydrocarbon-based chain, optionally containing one or more heteroatoms, optionally interrupted or substituted with one or more ether, C₁-C₂₀ alcohol ester, amine, carboxyl, C₆-C₃₀ aryl, hydroxyl or carbonyl groups, or a heterocyclic or non-heterocyclic aromatic ring, optionally substituted with one or more C₁-C₂₀ alcohol ester, amine, amide, carboxyl, hydroxyl, carbonyl or acyl groups.

Preferably, R₂₃ and R₂₃ₐ represent a hydrogen atom.

As explained previously, R₂₁, R₂₂, R₂₁ₐ and R₂₂ₐ each independently represent a hydrocarbon-based chain. The term "hydrocarbon-based chain" preferably means a chain comprising from 1 to 30 and preferably 1 to 10 carbon atoms.

Preferably, R₂₁ = R₂₁ₐ; R₂₂ = R₂₂ₐ; x = xa; y = ya; p = pa; B = Bₐ; q = 1 and qa = 0.

The alkoxysilane(s) of formula (II) may also have the following characteristics, taken alone or in combination:
- R₂₁, R₂₂, R₂₁ₐ and R₂₂ₐ, which may be identical or different, represent a C₁-C₄ alkyl,
- p = pa = 1;
- B and Bₐ, which may be identical or different, represent a linear C₁-C₄ alkylene.

For example, the alkoxysilane(s) are chosen from bis[3-(triethoxysilyl)propyl]amine of formula (CH₃CH₂O)₃-Si(CH₂)₃NH(CH₂)₃Si(OCH₂CH₃)₃ sold by the company Fluorochem, bis[trimethoxysilylpropyl]amine of formula (CH₃O)₃-Si(CH₂)₃NH(CH₂)₃Si(OCH₃)₃ sold by the company Gelest, bis[methyldiethoxysilylpropyl]amine of formula (CH₃CH₂O)₂CH₃Si(CH₂)₃NH(CH₂)₃SiCH₃(OCH₂CH₃)₂ sold by the company Gelest, and bis[3-trimethoxysilylpropyl]ethylenediamine of formula (CH₃O)₃Si(CH₂)₃NH(CH)₂NH(CH₂)₃Si(OCH₃)₃ sold by the company Gelest. Among these compounds, bis[3-(triethoxysilyl)propyl]amine and bis[methyldiethoxysilylpropyl]amine are preferred.

According to another embodiment of the invention, the alkoxysilane(s) are chosen from the compounds of formula (III): 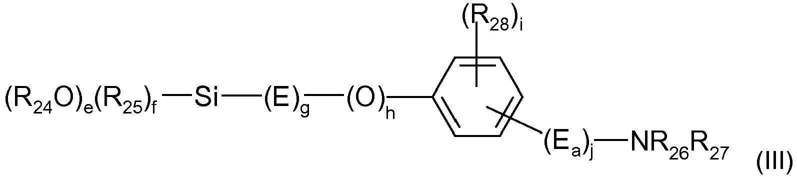 in which:
R₂₄ and R₂₅ represent, independently of each other, a saturated or unsaturated, linear or branched hydrocarbon-based chain, optionally containing one or more heteroatoms, optionally interrupted or substituted with one or more groups chosen from ether, ester, amine, amide, carboxyl, hydroxyl and carbonyl groups,
e = 2 or 3;
f = 3-e;
g = 0 or 1;
j = 0 or 1 ;
E and Eₐ each independently represent a linear or branched divalent C₁-C₂₀ alkylene radical,
R₂₆ and R₂₇ each independently represent a hydrogen atom or a saturated or unsaturated, linear or branched hydrocarbon-based chain, optionally containing one or more heteroatoms, optionally interrupted or substituted with one or more ether, C₁-C₂₀ alcohol ester, amine, carboxyl, C₆-C₃₀ aryl, hydroxyl or carbonyl groups, or a heterocyclic or non-heterocyclic aromatic ring, optionally substituted with one or more C₁-C₂₀ alcohol ester, amine, amide, carboxyl, hydroxyl, carbonyl or acyl groups,
i is an integer ranging from 0 to 4,
h is 0 or 1,
the group(s) R₂₈ each independently represent a hydrogen atom or a saturated or unsaturated, linear or branched, preferably C₁-C₁₀ hydrocarbon-based chain, optionally containing one or more heteroatoms, optionally interrupted or substituted with one or more ether, C₁-C₂₀ alcohol ester, amine, carboxyl, C₆-C₃₀ aryl, hydroxyl or carbonyl groups, or a heterocyclic or non-heterocyclic aromatic ring, optionally substituted with one or more C₁-C₂₀ alcohol ester, amine, amide, carboxyl, hydroxyl, carbonyl or acyl groups.

As explained previously, R₂₄ and R₂₅ each independently represent a hydrocarbon-based chain. The term "hydrocarbon-based chain" preferably means a chain comprising from 1 to 30 and preferably 1 to 10 carbon atoms.

Similarly, R₂₆ or R₂₇ may represent a hydrocarbon-based chain. In this case, it preferably means a chain comprising from 1 to 30 and preferably 1 to 10 carbon atoms.

Preferably, the aromatic ring comprises from 6 to 30 carbon atoms. Even more preferentially, it denotes an optionally substituted phenyl radical.

The alkoxysilane(s) of formula (III) may have the following characteristics, taken alone or in combination:
- R₂₄ is a C₁-C₄ alkyl,
- e=3,g=j=1;i=h=0,

R₂₆ and R₂₇ independently represent hydrogen or a group chosen from C₁-C₄ alkyl, C₁-C₄ hydroxyalkyl and C₁-C₄ aminoalkyl groups.

Preferably, R₂₆ or R₂₇ denote a hydrogen atom.

In particular, the alkoxysilane(s) of formula (III) may be chosen from:
- 3-(m-aminophenoxy)propyltrimethoxysilane, of formula: 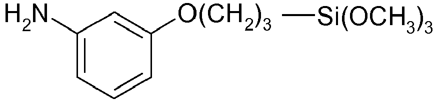
- p-aminophenyltrimethoxysilane, of formula: 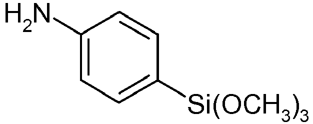
- N-(2-aminoethylaminomethyl)phenethyltrimethoxysilane, of formula:

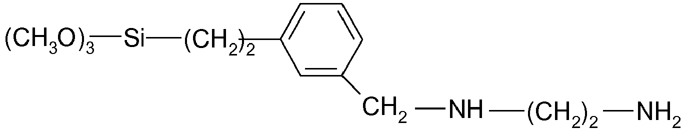

More preferentially, the alkoxysilane(s) that may be used in the compositions according to the present invention correspond to formula (IV): 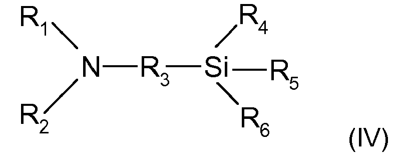 in which:
R₁ and R₂, independently of each other, are chosen from hydrogen and ethyl, propyl and aminoethyl groups;
R₃ is chosen from ethyl, propyl and methylphenethyl groups;
R₄, R₅ and R₆, independently of each other, are chosen from methyl, methoxy and ethoxy groups.

In one variant of the invention, the alkoxysilanes of the invention comprise one or more primary or secondary amine functions.

Preferably, the alkoxysilanes of the invention are chosen from the compounds of formulae (I), (III) and (IV) and more particularly the following compounds: 3-aminopropyltriethoxysilane (APTES), 3-aminoethyltriethoxysilane (AETES), 3-aminopropylmethyldiethoxysilane, N-(2-aminoethyl)-3-aminopropyltriethoxysilane and N-(2-aminoethylaminomethyl)phenethyl-trimethoxysilane of formula:

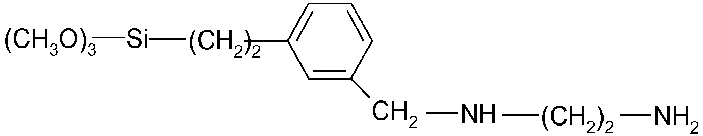

More particularly, the alkoxysilanes of the invention are chosen from the compounds of formula (I), especially 3-aminopropyltriethoxysilane (APTES), 3-aminoethyltriethoxysilane (AETES), 3-aminopropylmethyldiethoxysilane and N-(2-aminoethyl)-3-aminopropyltriethoxysilane and preferably the alkoxysilane is 3-aminopropyltriethoxysilane (APTES).

The alkoxysilane(s) may be present in the cosmetic composition according to the invention in a content ranging from 0.05% to 20%, in particular from 0.1% to 10%, preferably from 0.2% to 5%, relative to the total weight of the composition.

The composition according to the invention comprises one or more fatty esters.

For the purposes of the present invention, the term "fatty esters" more particularly means an ester of a carboxylic acid comprising in its structure a fatty chain with at least 10 carbon atoms, preferably having from 10 to 30 carbon atoms, preferably from 10 to 22 carbon atoms, and of an alcohol which is preferably a monoalcohol, especially C₁-C₃₀, more particularly C₁-C₂₂, or a sugar.

More particularly, these compounds are chosen from:
- esters of saturated, linear or branched C₁-C₃₀ monoalcohols, with C₁₀-C₃₀ monofunctional fatty acids, the latter possibly being linear or branched, and saturated or unsaturated;
- esters of linear or branched C₃-C₈ monoalcohols, with C₁₀-C₃₀ difunctional fatty acids, the latter possibly being linear or branched, and saturated or unsaturated;
- esters and diesters of sugars and of C₁₀-C₃₀ fatty acids;
- mixtures thereof.

As regards the esters of saturated, linear or branched C₁-C₃₀ monoalcohols, with C₁₀-C₃₀ monofunctional fatty acids, the latter may be linear or branched, and saturated or unsaturated. If they are unsaturated, these compounds may comprise one to three conjugated or unconjugated carbon-carbon double bonds (-C=C-).

According to one preferred embodiment of the invention, these esters may be selected especially from oleate, laurate, palmitate, myristate, behenate, cocoate, stearate, linoleate, linolenate, caprate, arachidonate, or mixtures thereof, especially such as the oleopalmitates, oleostearates and palmitostearates of C₁-C₃₀ monoalcohols.

Among these esters, use may be made of branched alcohol esters such as isopropyl palmitate, isopropyl myristate, octyldodecyl stearate and isononyl isononanoate. These esters are liquid at 25°C and at atmospheric pressure (10⁵ Pa).

Among the esters of linear or branched C₃-C₈ monoalcohols, with difunctional, linear or branched, saturated or unsaturated C₁₂-C₃₀ fatty acids, and more particularly among the isopropyl diester of sebacic acid, also known as diisopropyl sebacate.

The composition may also comprise, as fatty ester, sugar esters and diesters of C₁₀-C₃₀ fatty acids. It is recalled that the term "sugar" means compounds that have several alcohol functions, with or without aldehyde or ketone functions, and which comprise at least 4 carbon atoms. These sugars may be monosaccharides, oligosaccharides or polysaccharides.

Examples of suitable sugars that may be mentioned include sucrose (or saccharose), glucose, galactose, ribose, fucose, maltose, fructose, mannose, arabinose, xylose and lactose, and derivatives thereof, especially alkyl derivatives, such as methyl derivatives, for instance methylglucose.

The sugar esters of fatty acids may be selected especially from the group comprising the esters or mixtures of esters of sugars described previously and of linear or branched, saturated or unsaturated C₁₀-C₂₂ fatty acids. If they are unsaturated, these compounds may comprise one to three conjugated or nonconjugated carbon-carbon double bonds.

The esters may also be selected from monoesters, diesters, triesters, tetraesters and polyesters, and mixtures thereof.

These esters may be, for example, oleates, laurates, palmitates, myristates, behenates, cocoates, stearates, linoleates, linolenates, caprates and arachidonates of sugar(s), or mixtures thereof such as, especially, oleopalmitate, oleostearate and palmitostearate mixed esters of sugar(s).

More particularly, use is made of monoesters and diesters and especially sucrose, glucose or methylglucose monooleates or dioleates, stearates, behenates, oleopalmitates, linoleates, linolenates and oleostearates.

An example that may be mentioned is the product sold under the name Glucate® DO by the company Amerchol, which is a methylglucose dioleate.

Examples of esters or mixtures of esters of sugar and of fatty acid that may also be mentioned include:
- the products sold under the names F160, F140, F110, F90, F70 and SL40 by the company Crodesta, respectively denoting sucrose palmitostearates formed from 73% monoester and 27% diester and triester, from 61% monoester and 39% diester, triester and tetraester, from 52% monoester and 48% diester, triester and tetraester, from 45% monoester and 55% diester, triester and tetraester, from 39% monoester and 61% diester, triester and tetraester, and sucrose monolaurate;
- the products sold under the name Ryoto Sugar Esters, for example referenced B370 and corresponding to sucrose behenate formed from 20% monoester and 80% diester, triester and polyester;
- the sucrose monodipalmitostearate sold by the company Goldschmidt under the name Tegosoft^{®} PSE.

According to one particularly preferred embodiment, the fatty esters used in the composition of the invention are preferably saturated fatty esters, i.e. esters of saturated carboxylic acids comprising at least 10 carbon atoms, and of saturated fatty monoalcohols comprising at least 10 carbon atoms. The saturated acids or monoalcohols may be linear or branched. The saturated carboxylic acids preferably comprise from 10 to 30 carbon atoms and more preferentially from 12 to 24 carbon atoms. They may optionally be hydroxylated. The saturated fatty monoalcohols preferably comprise from 10 to 30 carbon atoms and more particularly from 12 to 24 carbon atoms.

Preferably, the carboxylic acids and alcohols of these particular esters are saturated and linear.

Preferably, the fatty esters are chosen from myristyl myristate, cetyl myristate, stearyl myristate, myristyl palmitate, cetyl palmitate, stearyl palmitate, myristyl stearate, cetyl stearate, stearyl stearate and behenyl behenate, and mixtures thereof. These esters are solid at 25°C and at atmospheric pressure (10⁵ Pa).

Preferably, the fatty esters of the invention are solid at 25°C and at atmospheric pressure (10⁵ Pa).

According to the invention, the fatty ester(s) may represent from 0.01% to 10% by weight, preferably from 0.1 % to 5% by weight and more particularly from 0.1 % to 5% by weight relative to the total weight of the composition.

The weight ratio of the amount of fatty ester to the amount of alkoxysilane(s) ranges from 0.005 to 20, preferably from 0.01 to 10 and better still from 0.02 to 5.

The silicones that may be used in the compositions of the present invention are in particular polyorganosiloxanes, which may be oils, waxes, resins or gums.

The silicones that may be used in the compositions of the present invention may be in the form of aqueous solutions, i.e. dissolved, or optionally in the form of dispersions or microdispersions, or of aqueous emulsions.

Silicones are defined in greater detail in Walter Noll's Chemistry and Technology of Silicones (1968), Academic Press.

The silicones may be volatile or non-volatile.

When they are volatile, the silicones are more particularly chosen from those with a boiling point of between 60°C and 260°C, and even more particularly from:
(i) cyclic silicones comprising from 3 to 7 and preferably 4 to 5 silicon atoms.
   These are, for example, octamethylcyclotetrasiloxane sold especially under the name Volatile Silicone 7207 by the company Union Carbide or Silbione 70045 V 2 by the company Rhodia, decamethylcyclopentasiloxane sold under the name Volatile Silicone 7158 by the company Union Carbide, and Silbione 70045 V 5 by the company Rhodia, and mixtures thereof.
   Mention may also be made of cyclocopolymers of the dimethylsiloxane/methylalkylsiloxane type, such as Volatile Silicone FZ 3109 sold by the company Union Carbide, of chemical structure: 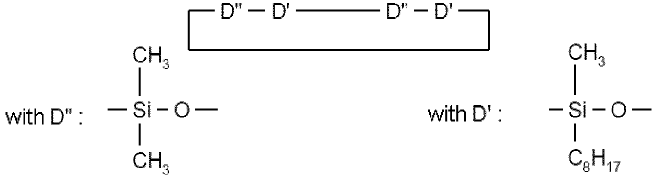
   Mention may also be made of mixtures of cyclic silicones with organosilicon compounds, such as the mixture of octamethylcyclotetrasiloxane and tetrakis(trimethylsilyl)pentaerythritol (50/50) and the mixture of octamethylcyclotetrasiloxane and oxy-1,1'-bis(2,2,2',2',3,3'-hexatrimethylsilyloxy)neopentane;
(ii) linear volatile silicones containing 2 to 9 silicon atoms and having a viscosity of less than or equal to 5×10⁻⁶ m²/s at 25°C. An example is decamethyltetrasiloxane sold in particular under the name SH 200 by the company Toray Silicone. Silicones belonging to this category are also described in the article published in Cosmetics and Toiletries, Vol. 91, Jan. 76, pp. 27-32, Todd & Byers, Volatile Silicone Fluids for Cosmetics*.*

When the silicones are non-volatile, use is preferably made of polyalkylsiloxanes, polyarylsiloxanes, polyalkylarylsiloxanes, silicone gums and resins, and polyorganosiloxanes modified with organofunctional groups, and mixtures thereof.

These silicones are more particularly chosen from polydialkylsiloxanes, among which mention may be made mainly of polydimethylsiloxanes containing trimethylsilyl end groups (Dimethicone according to the CTFA name) for example having a viscosity of from 5×10⁻⁶ to 2.5 m²/s at 25°C and preferably 1×10⁻⁵ to 1 m²/s. The viscosity of the silicones is measured, for example, at 25°C according to standard ASTM 445 Appendix C.

Among these polyalkylsiloxanes, mention may be made, in a non-limiting manner, of the following commercial products:
- the Silbione oils of the 70 047 series or the Mirasil oils sold by the company Rhodia, for instance the oil 70 047 V 500 000,
- the oils of the 200 series from the company Dow Corning, such as, more particularly, DC200 with a viscosity of 60 000 cSt,
- the Viscasil oils from the company General Electric and certain oils of the SF series (SF 96, SF 18) from the company General Electric.

Mention may also be made of polydialkylsiloxanes and especially polydimethylsiloxanes containing dimethylsilanol end groups (Dimethiconol according to the CTFA name) such as the oils of the 48 series from the company Rhodia.

Mention may also be made of polydimethylsiloxanes containing aminoethyl, aminopropyl and α,ω-silanol groups.

In this category of polyalkylsiloxanes, mention may also be made of the products sold under the names Abil Wax 9800 and 9801 by the company Goldschmidt, which are poly(C₁-C₂₀)alkylsiloxanes.

The polyalkylarylsiloxanes are particularly chosen from linear and/or branched polydimethylmethylphenylsiloxanes and polydimethyldiphenylsiloxanes with a viscosity of from 1×10⁻⁵ to 5×10⁻² m²/s at 25°C.

Among these polyalkylarylsiloxanes, examples that may be mentioned include the products sold under the following names:
- Silbione oils of the 70 641 series from the company Rhodia,
- the oils of the Rhodorsil 70 633 and 763 series from the company Rhodia,
- the oil Dow Corning 556 Cosmetic Grade Fluid from the company Dow Corning,
- the silicones of the PK series from the company Bayer, such as the product PK20,
- the silicones of the PN and PH series from the company Bayer, such as the products PN1000 and PH1000,
- certain oils of the SF series from the company General Electric, such as SF 1023, SF 1154, SF 1250 and SF 1265.

The silicone gums that may be present in the composition according to the invention are especially polydiorganosiloxanes having high number-average molecular masses of between 200 000 and 1 000 000, used alone or as a mixture in a solvent. This solvent can be chosen from volatile silicones, polydimethylsiloxane (PDMS) oils, polyphenylmethylsiloxane (PPMS) oils, isoparaffins, polyisobutylenes, methylene chloride, pentane, dodecane and tridecane, or mixtures thereof.

Mention may be made more particularly of the following products:
- polydimethylsiloxane gums,
- polydimethylsiloxane/methylvinylsiloxane gums,
- polydimethylsiloxane/diphenylsiloxane gums,
- polydimethylsiloxane/phenylmethylsiloxane gums,
- polydimethylsiloxane/diphenylsiloxane/methylvinylsiloxane gums. Products that may be used more particularly are the following mixtures:
- mixtures formed from a polydimethylsiloxane hydroxylated at the end of the chain (known as dimethiconol according to the nomenclature of the CTFA dictionary) and from a cyclic polydimethylsiloxane (known as cyclomethicone according to the nomenclature of the CTFA dictionary), such as the product Q2 1401 sold by the company Dow Corning,
- mixtures formed from a polydimethylsiloxane gum with a cyclic silicone, such as the product SF 1214 Silicone Fluid from the company General Electric, this product being an SF 30 gum corresponding to a dimethicone, having a number-average molecular weight of 500 000, dissolved in the oil SF 1202 Silicone Fluid corresponding to decamethylcyclopentasiloxane,
- mixtures of two PDMSs with different viscosities, and more particularly of a PDMS gum and a PDMS oil, such as the product SF 1236 from the company General Electric. The product SF 1236 is the mixture of a gum SE 30 defined above, with a viscosity of 20 m²/s and of an oil SF 96 with a viscosity of 5×10⁻⁶ m²/s. This product preferably comprises 15% of gum SE 30 and 85% of an oil SF 96.

The organopolysiloxane resins that may be present in the composition according to the invention are crosslinked siloxane systems containing the following units: R₂SiO_{2/2}, R₃SiO_{1/2}, RSiO_{3/2} and SiO_{4/2}, in which R represents a hydrocarbon group containing 1 to 16 carbon atoms or a phenyl group. Among these products, the ones that are particularly preferred are those in which R denotes a C₁-C₄ alkyl group, more particularly methyl, or a phenyl group.

Among these resins, mention may be made of the product sold under the name Dow Corning 593 or those sold under the names Silicone Fluid SS 4230 and SS 4267 by the company General Electric, which are silicones of dimethyl/trimethylsiloxane structure.

Mention may also be made of the trimethyl siloxysilicate type resins sold especially under the names X22-4914, X21-5034 and X21-5037 by the company Shin-Etsu.

The organomodified silicones that may be present in the composition according to the invention are silicones as defined above and comprising in their structure one or more organofunctional groups attached via a hydrocarbon-based group.

Among the organomodified silicones, mention may be made of polyorganosiloxanes comprising:
- polyethyleneoxy and/or polypropyleneoxy groups optionally comprising C₆-C₂₄ alkyl groups, such as the products known as dimethicone copolyol sold by the company Dow Corning under the name DC 1248 or the oils Silwet L 722, L 7500, L 77 and L 711 by the company Union Carbide, and the (C₁₂)alkylmethicone copolyol sold by the company Dow Corning under the name Q2 5200,
- thiol groups, such as the products sold under the names GP 72 A and GP 71 from the company Genesee,
- alkoxylated groups, such as the product sold under the name Silicone Copolymer F-755 by SWS Silicones and Abil Wax 2428, 2434 and 2440 by the company Goldschmidt,
- hydroxylated groups, such as the polyorganosiloxanes containing a hydroxyalkyl function, described in French patent application FR 2 589 476,
- acyloxyalkyl groups, for instance the polyorganosiloxanes described in patent US-A-4 957 732,
- anionic groups of the carboxylic acid type, for instance in the products described in patent EP 186 507 from the company Chisso Corporation, or of the alkylcarboxylic type, such as those present in the product X-22-3701E from the company Shin-Etsu; 2-hydroxyalkyl sulfonate; 2-hydroxyalkyl thiosulfate such as the products sold by the company Goldschmidt under the names Abil S201 and Abil S255,
- hydroxyacylamino groups, for instance the polyorganosiloxanes described in patent application EP 342 834. Mention may be made, for example, of the product Q2-8413 from the company Dow Corning.

Among the organomodified silicones, mention may also be made of amino silicones.

For the purposes of the present invention, the term "amino silicone" means any silicone comprising at least one primary, secondary or tertiary amine function or a quaternary ammonium group.

The amino silicones that may be used in the cosmetic composition according to the present invention are chosen from:
(a) the compounds corresponding to formula (V) below:

   (R')ₐ(T)₃₋ₐSi[OSi(T)₂]ₙ-[OSi(T)_{b}(R¹)_{2-b}]ₘ-OSi(T)₃₋ₐ-(R¹)ₐ (V)

   in which:
   T is a hydrogen atom or a phenyl, hydroxyl (-OH) or C₁-C₈ alkyl group, and preferably methyl, or a C₁-C₈ alkoxy, preferably methoxy,
   a denotes the number 0 or an integer from 1 to 3, and preferably 0,
   b denotes 0 or 1, and in particular 1,
   m and n are numbers such that the sum (n + m) can range especially from 1 to 2000 and in particular from 50 to 150, it being possible for n to denote a number from 0 to 1999 and in particular from 49 to 149, and for m to denote a number from 1 to 2000 and in particular from 1 to 10,
   R¹ is a monovalent group of formula -C_{q}H_{2q}L in which q is a number from 2 to 8 and L is an optionally quaternized amino group chosen from the following groups:
      - N(R²)-CH₂-CH₂-N(R²)₂;
      - N(R²)₂,
      - N⁺(R²)₃ Q⁻,
      - N⁺(R²)(H)₂ Q⁻,
      - N⁺(R²)₂HQ⁻,
      - N(R²)-CH₂-CH₂-N⁺(R²)(H)₂ Q⁻,
      in which R² may denote a hydrogen atom, a phenyl, a benzyl or a saturated monovalent hydrocarbon-based group, for example a C₁-C₂₀ alkyl group, and Q⁻ represents a halide ion, for instance fluoride, chloride, bromide or iodide.

In particular, the amino silicones corresponding to the definition of formula (V) are chosen from the compounds corresponding to formula (VI) below: 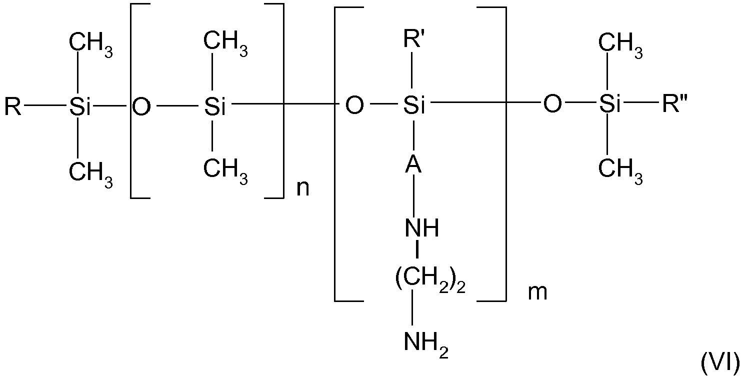 in which R, R' and R", which may be identical or different, denote a C₁-C₄ alkyl group, preferably CH₃; a C₁-C₄ alkoxy group, preferably methoxy; or OH; A represents a linear or branched, C₃-C₈ and preferably C₃-C₆ alkylene group; m and n are integers dependent on the molecular weight and whose sum is between 1 and 2000.

According to a first possibility, R, R' and R", which may be identical or different, represent a C₁-C₄ alkyl or hydroxyl group, A represents a C₃ alkylene group and m and n are such that the weight-average molecular mass of the compound is between 5000 and 500 000 approximately. Compounds of this type are referred to in the CTFA dictionary as "amodimethicones".

According to a second possibility, R, R' and R", which may be identical or different, each represent a C₁-C₄ alkoxy or hydroxyl group, at least one of the groups R or R" is an alkoxy group and A represents a C₃ alkylene group. The hydroxy/alkoxy mole ratio is preferably between 0.2/1 and 0.4/1 and advantageously equal to 0.3/1. Moreover, m and n are such that the weight-average molecular mass of the compound is between 2000 and 10⁶. More particularly, n is between 0 and 999 and m is between 1 and 1000, the sum of n and m being between 1 and 1000.

In this category of compounds, mention may be made, inter alia, of the product Belsil® ADM 652 sold by the company Wacker.

According to a third possibility, R and R", which are different, each represent a C₁-C₄ alkoxy or hydroxyl group, at least one of the groups R or R" being an alkoxy group, R' representing a methyl group and A representing a C₃ alkylene group. The hydroxy/alkoxy molar ratio is preferably between 1/0.8 and 1/1.1 and advantageously is equal to 1/0.95. Moreover, m and n are such that the weight-average molecular weight of the compound is between 2000 and 200 000. More particularly, n is between 0 and 999 and m is between 1 and 1000, the sum of n and m being between 1 and 1000.

More particularly, mention may be made of the product Fluid WR® 1300 sold by the company Wacker.

Note that the molecular mass of these silicones is determined by gel permeation chromatography (ambient temperature, polystyrene standard, µ styragem columns, eluent THF, flow rate of 1 mm/minute, 200 µl of a solution containing 0.5% by weight of silicone in THF are injected, and detection is performed by refractometry and UV-metry).

A product corresponding to the definition of formula (V) is in particular the polymer known in the CTFA dictionary as Trimethylsilyl Amodimethicone, corresponding to formula (VII) below: 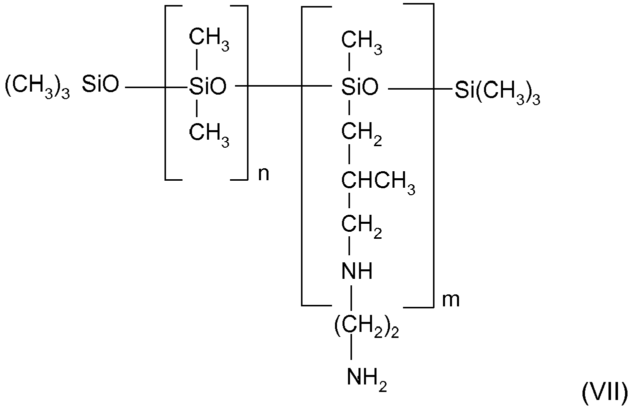 in which n and m have the meanings given above in accordance with formula (VI).

Such compounds are described, for example, in patent EP 95238. A compound of formula (VII) is sold, for example, under the name Q2-8220 by the company OSI.
(b) the compounds corresponding to formula (VIII) below: 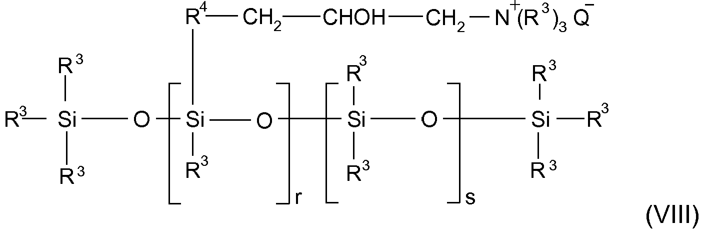 in which:
   R³ represents a C₁-C₁₈ monovalent hydrocarbon-based group, and in particular a C₁-C₁₈ alkyl or C₂-C₁₈ alkenyl group, for example methyl,
   R⁴ represents a divalent hydrocarbon-based group, especially a C₁-C₁₈ alkylene group or a divalent C₁-C₁₈, and for example C₁-C₈, alkylenoxy group,
   Q⁻ is a halide ion, in particular chloride,
   r represents a mean statistical value from 2 to 20 and in particular from 2 to 8;
   s represents a mean statistical value from 20 to 200 and in particular from 20 to 50.

Such compounds are described more particularly in patent US 4 185 087.

A compound falling within this class is the product sold by the company Union Carbide under the name Ucar Silicone ALE 56.
(c) quaternary ammonium silicones especially of formula (IX) below: 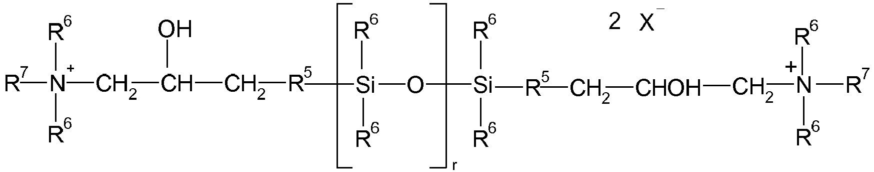 in which:
   R⁶, which may be identical or different, represent a monovalent hydrocarbon-based group containing from 1 to 18 carbon atoms, and in particular a C₁-C₁₈ alkyl group, a C₂-C₁₈ alkenyl group or a ring comprising 5 or 6 carbon atoms, for example methyl,
   R⁵ represents a divalent hydrocarbon-based group, especially a C₁-C₁₈ alkylene group or a divalent C₁-C₁₈, and for example C₁-C₈, alkylenoxy group linked to the Si via an Si-C bond,
   R⁷, which may be identical or different, represent a hydrogen atom, a monovalent hydrocarbon-based group containing from 1 to 18 carbon atoms, and in particular a C₁-C₁₈ alkyl group, a C₂-C₁₈ alkenyl group or a group -R⁵-NHCOR⁶;
   X⁻ is an anion such as a halide ion, especially chloride, or an organic acid salt (acetate, etc.);
   r represents a mean statistical value from 2 to 200 and in particular from 5 to 100.

These silicones are described, for example, in patent application EP-A-0 530 974.
d) the amino silicones of formula (X) below: 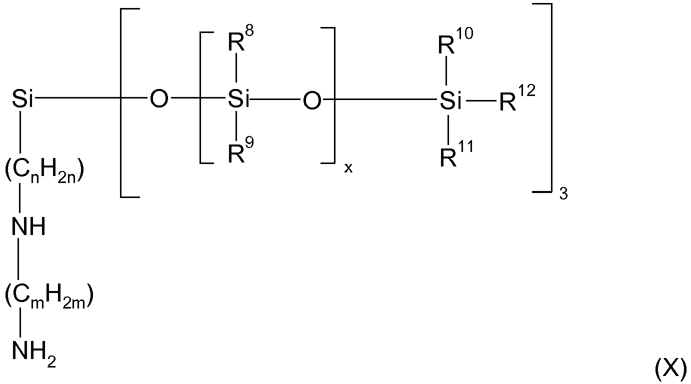 in which:
   - R⁸, R⁹, R¹⁰ and R¹¹, which may be identical or different, denote a C₁-C₄ alkyl group or a phenyl group,
   - R¹² denotes a C₁-C₄ alkyl group or a hydroxyl group,
   - n is an integer ranging from 1 to 5,
   - m is an integer ranging from 1 to 5, and
   - x is chosen such that the amine number is between 0.01 and 1 meq/g.

When these compounds are used, one particularly advantageous embodiment involves their combined use with cationic and/or nonionic surfactants.

By way of example, use may be made of the product sold under the name Cationic Emulsion DC939 by the company Dow Corning, a cationic surfactant, namely trimethylcetylammonium chloride and a nonionic surfactant of formula C₁₃H₂₇-(OC₂H₄)₁₂-OH, known under the CTFA name Trideceth-12.

Another commercial product that may be used according to the invention is the product sold under the name Dow Corning Q2 7224 by the company Dow Corning, comprising, in combination, trimethylsilyl amodimethicone of formula (X) described above, a nonionic surfactant of formula C₈H₁₇-C₆H₄-(OCH₂CH₂)₄₀-OH, known under the CTFA name Octoxynol-40, a second nonionic surfactant of formula C₁₂H₂₅-(OCH₂-CH₂)₆-OH, known under the CTFA name Isolaureth-6, and propylene glycol.

The silicones of the invention may also be silicones grafted with anionic groups, such as the compounds VS 80 or VS 70 sold by the company 3M.

According to the invention, all the silicones can also be used in the form of emulsions, nanoemulsions or microemulsions.

The particularly preferred silicones in accordance with the invention are:
- non-volatile silicones selected from the family of polydialkylsiloxanes with trimethylsilyl end groups, such as oils having a viscosity of between 0.2 and 2.5 m²/s at 25°C, for instance the oils of the DC200 series from Dow Corning, in particular the one with a viscosity of 60 000 cSt, or of the Silbione 70047 and 47 series, and more particularly the oil 70 047 V 500 000 sold by the company Rhodia Chimie, and polydialkylsiloxanes with dimethylsilanol end groups, such as dimethiconols, or polyalkylarylsiloxanes, for instance the oil Silbione 70641 V 200 sold by the company Rhodia Chimie;
- polysiloxanes bearing amine groups such as amodimethicones, trimethylsilyl amodimethicones and polysiloxanes bearing quaternary ammonium groups.

The silicones that are even more particularly preferred in accordance with the invention are polysiloxanes bearing amine groups.

The viscosities of the silicones may especially be determined by the standard ASTM D445-97 (viscometry).

The silicone(s) may represent from 0.01% to 10% by weight, preferably from 0.1 % to 5% by weight, more particularly from 0.3% to 5% by weight, and more particularly from 0.3% to 3% by weight, relative to the total weight of the composition.

The weight ratio of the amount of silicone(s) to the amount of alkoxysilane(s) ranges from 0.05 to 20, preferably from 0.1 to 10 and better still from 0.15 to 5.

The composition according to the invention may comprise water, one or more organic solvents or a mixture of water and one or more organic solvents, the organic solvents preferably being selected from C₁-C₄ lower alcohols such as ethanol, isopropanol, tert-butanol or n-butanol; polyols such as glycerol, propylene glycol and polyethylene glycols; and mixtures thereof.

In the composition according to the invention, the water may be present in a content ranging from 10% to 95% by weight and preferably ranging from 20% to 95% by weight relative to the total weight of the composition.

The composition according to the invention may also comprise one or more thickeners.

The thickener(s) may be selected from fatty acid amides obtained from a C₁₀-C₃₀ carboxylic acid (coconut acid monoisopropanolamide, diethanolamide or monoethanolamide, oxyethylenated alkyl ether carboxylic acid monoethanolamide), cellulose-based thickeners (hydroxyethyl cellulose, hydroxypropyl cellulose, carboxymethyl cellulose), guar gum and nonionic derivatives thereof (hydroxypropyl guar), gums of microbial origin (xanthan gum, scleroglucan gum), starches and associative polymers.

The associative polymer(s) that may be used according to the invention are water-soluble polymers which, in an aqueous medium, are capable of reversible association with each other or with other molecules.

Their chemical structure comprises hydrophilic zones, and hydrophobic zones characterized by at least one fatty chain containing preferably from 10 to 30 carbon atoms.

The associative polymer(s) that may be used according to the invention and that are different from the acrylic thickening polymers may be of anionic, cationic, amphoteric or nonionic type, such as the polymers sold under the names Elfacos T210 or T212 by the company Akzo.

Among all the additional thickeners mentioned, the thickener(s) are preferably chosen from cellulose-based thickeners.

When they are present, the composition preferably comprises from 0.1% to 20% by weight, and better still from 0.2% to 10% by weight, of additional thickener(s), relative to the total weight of the composition.

The composition according to the invention may also comprise one or more conditioning agents other than silicones.

According to the present invention, the term "conditioning agent" denotes any compound that can improve the cosmetic properties of the hair, in particular the softness, disentangling, feel and static electricity.

Preferably, the conditioning agent is chosen from the group comprising cationic polymers, cationic surfactants, linear or branched C₈-C₃₀ hydrocarbons, linear or branched C₈-C₃₀ fatty alcohols, ceramides or ceramide analogues, and mixtures of these compounds.

The term "cationic polymer" means a polymer that is positively charged when it is contained in the composition according to the invention. This polymer may bear one or more positive permanent charges or may contain one or more cationizable functions in the composition according to the invention.

The cationic polymer(s) that may be used as conditioning agents according to the present invention are preferably selected from polymers comprising primary, secondary, tertiary and/or quaternary amine groups forming part of the polymer chain or directly attached thereto, and having a molecular weight of between 500 and about 5 000 000 and preferably between 1000 and 3 000 000.

Among the cationic polymers that may be mentioned more particularly are polymers of the polyamine, polyaminoamide and polyquaternary ammonium type. These are known products. They are described, for example, in French Patents 2 505 348 and 2 542 997.

Among these polymers, mention may be made of:
(1) homopolymers or copolymers derived from acrylic or methacrylic esters or amides and comprising at least one of the units of the following formulae: 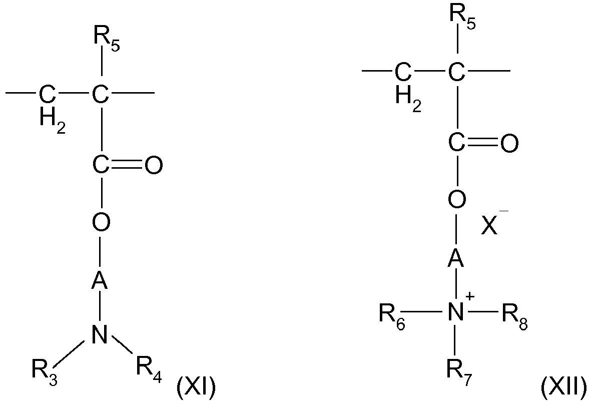 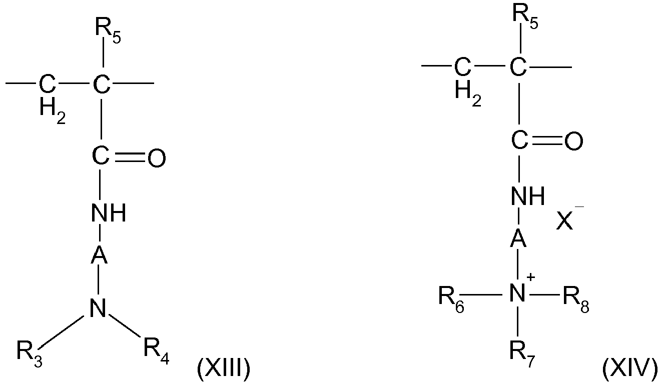 in which:
   R₃ and R₄, which are identical or different, represent a hydrogen atom or an alkyl group having from 1 to 6 carbon atoms, and preferably methyl or ethyl;
   R₅, which are identical or different, denote a hydrogen atom or a CH₃ group;
   A, which are identical or different, represent a linear or branched alkyl group having from 1 to 6 carbon atoms, preferably 2 or 3 carbon atoms, or a hydroxyalkyl group having from 1 to 4 carbon atoms;
   R₆, R₇, R₈, which are identical or different, represent an alkyl group having from 1 to 18 carbon atoms or a benzyl group, and preferably an alkyl group having from 1 to 6 carbon atoms;
   X⁻ denotes an anion derived from an organic or inorganic acid, such as a methosulfate anion, or a halide such as chloride or bromide.

   The copolymers of the family (1) may further contain one or more units deriving from comonomers which may be selected from the family of acrylamides, methacrylamides, diacetone acrylamides, acrylamides and methacrylamides substituted on the nitrogen by lower alkyls (C₁-C₄), acrylic acids or methacrylic acids or esters thereof, vinyllactams such as vinylpyrrolidone or vinylcaprolactam, and vinyl esters.
   Thus, among these copolymers of the family (1), mention may be made of:
   - copolymers of acrylamide and of dimethylaminoethyl methacrylate quaternized with dimethyl sulfate or with a dimethyl halide, such as that sold under the name Hercofloc by the company Hercules,
   - copolymers of acrylamide and of methacryloyloxyethyltrimethylammonium chloride, described, for example, in Patent Application EP-A-080 976 and sold under the name Bina Quat P 100 by the company Ciba Geigy,
   - the copolymer of acrylamide and of methacryloyloxyethyltrimethylammonium methosulfate sold under the name Reten by the company Hercules,
   - quaternized or non-quaternized vinylpyrrolidone/dialkylaminoalkyl acrylate or methacrylate copolymers, such as the products sold under the name Gafquat by the company ISP, such as, for example, Gafquat 734 or Gafquat 755, or alternatively the products known as Copolymer 845, 958 and 937. These polymers are described in detail in French Patents 2 077 143 and 2 393 573,
   - dimethylaminoethyl methacrylate/vinylcaprolactam/vinylpyrrolidone terpolymers, such as the product sold under the name Gaffix VC 713 by the company ISP,
   - vinylpyrrolidone/methacrylamidopropyldimethylamine copolymers sold in particular under the name Styleze CC 10 by the company ISP,
   - quaternized vinylpyrrolidone/dimethylaminopropyl methacrylamide copolymers, such as the product sold under the name Gafquat HS 100 by the company ISP, and
   - the crosslinked polymers of methacryloyloxy(C₁-C₄)alkyl tri(C₁-C₄)alkylammonium salts, such as the polymers obtained by homopolymerization of dimethylaminoethyl methacrylate quaternized with methyl chloride, or by copolymerization of acrylamide with dimethylaminoethyl methacrylate quaternized with methyl chloride, the homo- or copolymerization being followed by crosslinking with an olefinically unsaturated compound, more particularly methylenebisacrylamide. A crosslinked acrylamide/methacryloyloxyethyl-trimethylammonium chloride copolymer (20/80 by weight) in the form of a dispersion containing 50% by weight of said copolymer in mineral oil can be used more particularly. This dispersion is sold under the name Salcare® SC 92 by the company Ciba. A crosslinked methacryloyloxyethyltrimethylammonium chloride homopolymer, for example as a dispersion in mineral oil or in a liquid ester, can also be used. These dispersions are sold under the names Salcare® SC 95 and Salcare® SC 96 by the company Ciba.
(2) Polymers composed of piperazinyl units and of divalent alkylene or hydroxyalkylene groups containing straight or branched chains, optionally interrupted by oxygen, sulfur or nitrogen atoms or by aromatic or heterocyclic rings, and also the oxidation and/or quaternization products of these polymers. Such polymers are especially described in French Patents 2 162 025 and 2 280 361.
(3) Water-soluble polyaminoamides prepared in particular by polycondensation of an acidic compound with a polyamine; these polyaminoamides can be crosslinked with an epihalohydrin, a diepoxide, a dianhydride, an unsaturated dianhydride, a bis-unsaturated derivative, a bis-halohydrin, a bis-azetidinium, a bis-haloacyldiamine, a bis-alkyl halide or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent being used in proportions ranging from 0.025 to 0.35 mol per amine group of the polyaminoamide; these polyaminoamides can be alkylated or, if they comprise one or more tertiary amine functions, they can be quaternized. Such polymers are especially described in French Patents 2 252 840 and 2 368 508.
(4) Polyaminoamide derivatives resulting from the condensation of polyalkylene polyamines with polycarboxylic acids followed by alkylation with difunctional agents. Mention may be made, for example, of adipic acid/dialkylaminohydroxyalkyldialkylenetriamine polymers in which the alkyl groups contain from 1 to 4 carbon atoms and preferably denote a methyl, ethyl or propyl group, and the alkylene groups contain from 1 to 4 carbon atoms and preferably denote the ethylene group. Such polymers are especially described in French Patent 1 583 363.
   Among these derivatives, mention may be made more particularly of the adipic acid/dimethylaminohydroxypropyl/diethylenetriamine polymers sold under the name Cartaretine F, F4 or F8 by the company Sandoz.
(5) The polymers obtained by reaction of a polyalkylene polyamine containing two primary amine groups and at least one secondary amine group with a dicarboxylic acid selected from diglycolic acid and saturated aliphatic dicarboxylic acids having from 3 to 8 carbon atoms. The molar ratio between the polyalkylene polyamine and the dicarboxylic acid is between 0.8:1 and 1.4:1; the polyaminoamide resulting therefrom is reacted with epichlorohydrin in a molar ratio of epichlorohydrin relative to the secondary amine group of the polyaminoamide of between 0.5:1 and 1.8:1. Such polymers are described in particular in US patents 3 227 615 and 2 961 347.
   Polymers of this type are sold in particular under the name Hercosett 57 by the company Hercules Inc. or alternatively under the name PD 170 or Delsette 101 by the company Hercules in the case of the adipic acid/epoxypropyl/diethylenetriamine copolymer.
(6) Cyclopolymers of alkyldiallylamine or of dialkyldiallylammonium, such as the homopolymers or copolymers containing, as main constituent of the chain, units corresponding to formula (XV) or (XVI): 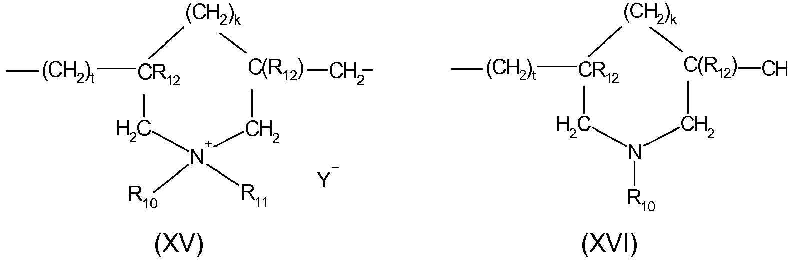 in which formulae: k and t are equal to 0 or 1, the sum k + t being equal to 1; R₁₂ denotes a hydrogen atom or a methyl group; R₁₀ and R₁₁, independently of one another, denote an alkyl group having from 1 to 6 carbon atoms, a hydroxyalkyl group in which the alkyl group has preferably 1 to 5 carbon atoms, a lower (C₁-C₄) amidoalkyl group, or else R₁₀ and R₁₁ may, together with the nitrogen atom to which they are attached, denote heterocyclic groups, such as piperidyl or morpholinyl; Y⁻ is an anion such as bromide, chloride, acetate, borate, citrate, tartrate, bisulfate, bisulfite, sulfate or phosphate. These polymers are especially described in French Patent 2 080 759 and in its Certificate of Addition 2 190 406.
   R₁₀ and R₁₁, independently of one another, denote preferably an alkyl group having from 1 to 4 carbon atoms.
   Among the polymers defined above, mention may be made more particularly of the dimethyldiallylammonium chloride homopolymer sold under the name Merquat 100 by the company Nalco (and its homologues of low weight-average molecular weights) and the copolymers of diallyldimethylammonium chloride and of acrylamide, sold under the name Merquat 550.
(7) The quaternary diammonium polymer containing repeating units corresponding to the formula (XVII): 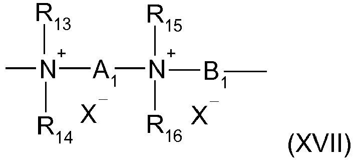 in which formula (XV):
   R₁₃, R₁₄, R₁₅ and R₁₆, which may be identical or different, represent aliphatic, alicyclic or arylaliphatic groups containing from 1 to 7 carbon atoms or lower (C₁-C₄) hydroxyalkylaliphatic groups, or alternatively R₁₃, R₁₄, R₁₅ and R₁₆, together or separately, constitute, with the nitrogen atoms to which they are attached, heterocycles optionally containing a second heteroatom other than nitrogen, or alternatively R₁₃, R₁₄, R₁₅ and R₁₆ represent a linear or branched C₁-C₆ alkyl group substituted with a nitrile, ester, acyl or amide group or a -CO-O-R₁₇-D or -CO-NH-R₁₇-D group where R₁₇ is an alkylene having from 1 to 10 carbon atoms and D is a quaternary ammonium group;
   A₁ and B₁ represent polymethylene groups containing from 2 to 20 carbon atoms, which may be linear or branched and saturated or unsaturated and may contain, joined to or intercalated in the main chain, one or more aromatic rings, or one or more oxygen or sulfur atoms or sulfoxide, sulfone, disulfide, amino, alkylamino, hydroxyl, quaternary ammonium, ureido, amide or ester groups, and
   X⁻ denotes an anion derived from an inorganic or organic acid;
   A₁, R₁₃ and R₁₅ may, with the two nitrogen atoms to which they are attached, form a piperazine ring; moreover, if A₁ denotes a linear or branched, saturated or unsaturated alkylene or hydroxyalkylene group, B₁ may also denote a group

      -(CH₂)ₙ-CO-D-OC-(CH₂)ₚ-

      in which:
      n and p are integers ranging from 2 to 20 approximately,
      D denotes:
         a) a glycol residue of formula: -O-Z-O-, where Z denotes a linear or branched hydrocarbon-based group or a group corresponding to one of the following formulae:

            -(CH₂-CH₂-O)ₓ -CH₂-CH₂-

            -[CH₂-CH(CH₃)-O]_{y}-CH₂-CH(CH₃)-

            where x and y denote an integer from 1 to 4, representing a defined and unique degree of polymerization or any number from 1 to 4 representing an average degree of polymerization;
         b) a bis-secondary diamine residue such as a piperazine derivative;
         c) a bis-primary diamine residue of formula: -NH-Y-NH-, where Y denotes a linear or branched hydrocarbon-based group, or else the divalent group -CH₂-CH₂-S-S-CH₂-CH₂-;
         d) a ureylene group of formula: -NH-CO-NH-.

   Preferably, X⁻ is an anion such as chloride or bromide.
   These polymers generally have a number-average molecular weight of between 1000 and 100 000.
   Polymers of this type are described in particular in French patents 2 320 330, 2 270 846, 2 316 271, 2 336 434 and 2 413 907 and US patents 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 and 4 027 020.
   Use may more particularly be made of the polymers which are composed of repeating units corresponding to the formula (XVIII): 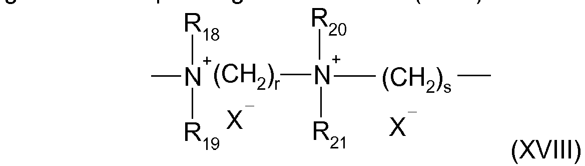 in which: R₁₈, R₁₉, R₂₀ and R₂₁, which are identical or different, denote an alkyl or hydroxyalkyl group having from 1 to 4 carbon atoms approximately, r and s are integers ranging from 2 to 20 approximately, and X⁻ is an anion derived from an inorganic or organic acid.
   One particularly preferred compound of formula (XVIII) is that for which R₁₈, R₁₉, R₂₀ and R₂₁ represent a methyl group and r = 3, s = 6 and X = Cl, which is called Hexadimethrine chloride according to INCI nomenclature (CTFA).
(8) Polyquaternary ammonium polymers composed of units of formula (XIX): 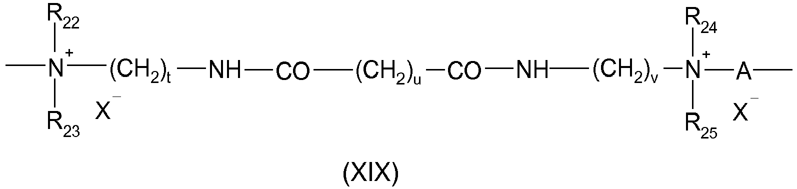 in which formula:
   R₂₂, R₂₃, R₂₄ and R₂₅, which are identical or different, represent a hydrogen atom or a methyl, ethyl, propyl, β-hydroxyethyl, β-hydroxypropyl or - CH₂CH₂(OCH₂CH₂)ₚOH group, where p is equal to 0 or to an integer between 1 and 6, with the proviso that R₂₂, R₂₃, R₂₄ and R₂₅ do not simultaneously represent a hydrogen atom,
   t and u, which may be identical or different, are integers between 1 and 6,
   v is equal to 0 or to an integer between 1 and 34,
   X⁻ denotes an anion such as a halide,
   A denotes a dihalide group or preferably represents -CH₂-CH₂-O-CH₂-CH₂-.

   Such compounds are described especially in Patent Application EP-A-122 324.
   Among these, mention may be made, for example, of the products Mirapol® A 15, Mirapol® AD1, Mirapol® AZ1 and Mirapol® 175, sold by the company Miranol.
(9) Quaternary polymers of vinylpyrrolidone and of vinylimidazole, for instance the products sold under the names Luviquat® FC 905, FC 550 and FC 370 by the company BASF.
(10) Cationic polysaccharides, in particular cationic celluloses and derivatives of cationic celluloses, and cationic galactomannan gums.

Among the cationic polysaccharides, mention may be made more particularly of cellulose ether derivatives comprising quaternary ammonium groups, cationic cellulose copolymers or cellulose derivatives grafted with a water-soluble quaternary ammonium monomer and cationic galactomannan gums.

The cellulose ether derivatives comprising quaternary ammonium groups are described in French Patent 1 492 597. These polymers are also defined in the CTFA dictionary as quaternary ammoniums of hydroxyethyl cellulose that have reacted with an epoxide substituted with a trimethylammonium group.

The cationic cellulose copolymers or the cellulose derivatives grafted with a water-soluble quaternary ammonium monomer are described especially in Patent US 4 131 576, such as hydroxyalkyl celluloses, for instance hydroxymethyl, hydroxyethyl or hydroxypropyl celluloses grafted especially with a methacryloylethyltrimethylammonium, methacrylamidopropyltrimethyl-ammonium or dimethyldiallylammonium salt.

The cationic galactomannan gums are described more particularly in US patents 3 589 578 and 4 031 307, in particular guar gums containing cationic trialkylammonium groups. Use is made, for example, of guar gums modified with a 2,3-epoxypropyltrimethylammonium salt (for example, chloride).

Other cationic polymers that may be used in the context of the invention are cationic proteins or cationic protein hydrolysates, polyalkyleneimines, in particular polyethyleneimines, polymers containing vinylpyridine or vinylpyridinium units, condensates of polyamines and of epichlorohydrin, quaternary polyureylenes and chitin derivatives.

The cationic proteins or protein hydrolysates are, in particular, chemically modified polypeptides bearing quaternary ammonium groups at the end of the chain, or grafted thereon. Their molecular weight may vary, for example, from 1500 to 10 000 and in particular from 2000 to 5000 approximately. Among these compounds, mention may be made especially of:
- collagen hydrolysates bearing triethylammonium groups, such as the products sold under the name Quat-Pro E by the company Maybrook and referred to in the CTFA dictionary as Triethonium Hydrolyzed Collagen Ethosulfate;
- collagen hydrolysates bearing trimethylammonium chloride and trimethylstearylammonium chloride groups, which are sold under the name Quat-Pro S by the company Maybrook and are referred to in the CTFA dictionary as Steartrimonium Hydrolyzed Collagen;
- animal protein hydrolysates bearing trimethylbenzylammonium groups, such as the products sold under the name Crotein BTA by the company Croda and referred to in the CTFA dictionary as Benzyltrimonium hydrolyzed animal protein;
- protein hydrolysates bearing quaternary ammonium groups on the polypeptide chain, said ammonium groups containing at least one alkyl group having from 1 to 18 carbon atoms.

Among these protein hydrolysates, mention may be made, inter alia, of:
- Croquat L, in which the quaternary ammonium groups contain a C₁₂ alkyl group;
- Croquat M, in which the quaternary ammonium groups contain C₁₀-C₁₈ alkyl groups;
- Croquat S, in which the quaternary ammonium groups contain a C₁₈ alkyl group;
- Crotein Q, in which the quaternary ammonium groups contain at least one alkyl group having from 1 to 18 carbon atoms.

These various products are sold by the company Croda.

Other quaternized proteins or hydrolysates are, for example, those corresponding to the formula (XX): 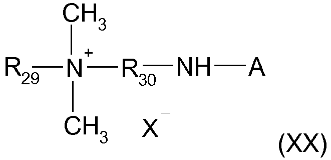 in which X⁻ is an anion of an organic or inorganic acid, A denotes a protein residue derived from collagen protein hydrolysates, R₂₉ denotes a lipophilic group containing up to 30 carbon atoms, and R₃₀ represents an alkylene group having 1 to 6 carbon atoms. Mention may, for example, be made of the products sold by the company Inolex, under the name Lexein QX 3000, called, in the CTFA dictionary, Cocotrimonium Collagen Hydrolysate.

Mention may also be made of quaternized plant proteins such as wheat, corn or soybean proteins: quaternized wheat proteins that may be mentioned include those sold by the company Croda under the names Hydrotriticum WQ or QM, which in the CTFA dictionary are called Cocodimonium Hydrolysed wheat protein, or Hydrotriticum QL, which in the CTFA dictionary is called Laurdimonium hydrolysed wheat protein, or else Hydrotriticum QS, which in the CTFA dictionary is called Steardimonium hydrolysed wheat protein.

Among all the cationic polymers that may be used in the context of the present invention, it is preferred to use cationic cyclopolymers, such as defined above, in particular the dimethyldiallylammonium chloride homopolymers or copolymers sold under the names Merquat 100, Merquat 550 and Merquat S by the company Nalco, and quaternary vinylpyrrolidone and vinylimidazole polymers, cationic polysaccharides and mixtures thereof.

The conditioning agent(s) that can be used according to the invention may be selected from cationic surfactants.

The term "cationic surfactant" means a surfactant that is positively charged when it is contained in the composition according to the invention. This surfactant may bear one or more positive permanent charges or may contain one or more functions that are cationizable in the composition according to the invention.

The cationic surfactant(s) that may be used as conditioning agents according to the present invention are preferably selected from optionally polyoxyalkylenated primary, secondary or tertiary fatty amines, or the salts thereof, quaternary ammonium salts, and mixtures thereof.

The fatty amines generally comprise at least one C₈-C₃₀ hydrocarbon-based chain. Among the fatty amines that may be used according to the invention, examples that may be mentioned include stearylamidopropyldimethylamine and distearylamine.

Examples of quaternary ammonium salts that may especially be mentioned include:
- those corresponding to the general formula (XXI) below: 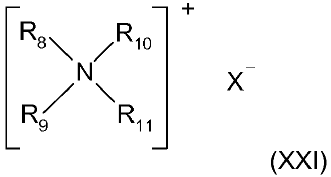 in which the groups R₈ to R₁₁, which may be identical or different, represent a linear or branched aliphatic group containing from 1 to 30 carbon atoms, or an aromatic group such as aryl or alkylaryl, at least one of the groups R₈ to R₁₁ denoting a group containing from 8 to 30 carbon atoms, preferably from 12 to 24 carbon atoms. The aliphatic groups may comprise heteroatoms especially such as oxygen, nitrogen, sulfur and halogens. The aliphatic groups are selected, for example, from C₁₋₃₀ alkyl, C₁-₃₀ alkoxy, polyoxyalkylene (C₂-C₆), C₁-₃₀ alkylamide, (C₁₂-C₂₂)alkylamido(C₂-C₆)alkyl, (C₁₂-C₂₂)alkylacetate, and C₁₋₃₀ hydroxyalkyl groups; X⁻ is an anion selected from the group of halides, phosphates, acetates, lactates, (C₁-C₄)alkyl sulfates and (C₁-C₄)alkyl sulfonates or (C₁-C₄)alkylaryl sulfonates.

Among the quaternary ammonium salts of formula (XIX), those that are preferred are, on the one hand, tetraalkylammonium salts, for instance dialkyldimethylammonium or alkyltrimethylammonium salts in which the alkyl group contains approximately from 12 to 22 carbon atoms, in particular behenyltrimethylammonium, distearyldimethylammonium, cetyltrimethylammonium or benzyldimethylstearylammonium salts, or, on the other hand, the palmitylamidopropyltrimethylammonium salt, the stearamidopropyltrimethylammonium salt, the stearamidopropyldimethyl-cetearylammonium salt, or the stearamidopropyldimethyl(myristyl acetate)ammonium salt sold under the name Ceraphyl® 70 by the company Van Dyk. It is particularly preferred to use the chloride salts of these compounds;
- quaternary ammonium salts of imidazoline, such as, for example, those of formula (XXII) below: 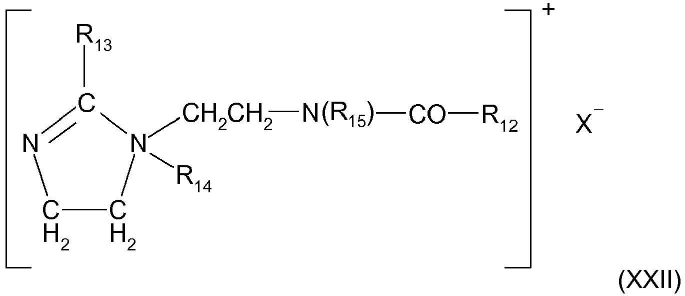 in which R₁₂ represents an alkenyl or alkyl group containing from 8 to 30 carbon atoms, for example tallow fatty acid derivatives, R₁₃ represents a hydrogen atom, a C₁-C₄ alkyl group or an alkenyl or alkyl group containing from 8 to 30 carbon atoms, R₁₄ represents a C₁-C₄ alkyl group, R₁₅ represents a hydrogen atom or a C₁-C₄ alkyl group, X⁻ is an anion selected from the group of halides, phosphates, acetates, lactates, alkyl sulfates, alkyl sulfonates or alkylaryl sulfonates, the alkyl and aryl groups of which preferably comprise, respectively, from 1 to 20 carbon atoms and from 6 to 30 carbon atoms. R₁₂ and R₁₃ preferably denote a mixture of alkenyl or alkyl groups comprising from 12 to 21 carbon atoms, for example tallow fatty acid derivatives, R₁₄ denotes a methyl group, and R₁₅ denotes a hydrogen atom. Such a product is sold, for example, under the name Rewoquat® W 75 by the company Rewo;
- quaternary di- or triammonium salts of formula (XXIII): 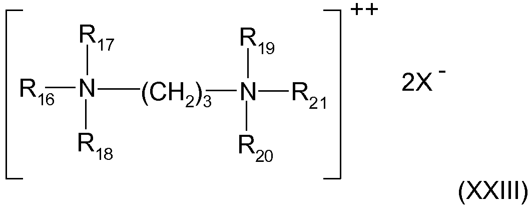 in which R₁₆ denotes an alkyl group containing approximately from 16 to 30 carbon atoms, which is optionally hydroxylated and/or interrupted with one or more oxygen atoms, R₁₇ is selected from hydrogen or an alkyl group containing from 1 to 4 carbon atoms or the following group: 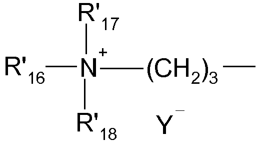 R'₁₆, R'₁₇, R'₁₈, R₁₈, R₁₉, R₂₀ and R₂₁, which may be identical or different, are selected from hydrogen or an alkyl group containing from 1 to 4 carbon atoms, and X⁻ and Y⁻ are anions in particular selected from the group of halides, acetates, phosphates, nitrates and (C₁-C₆)alkyl sulfates, in particular methyl sulfate or ethyl sulfate. Such compounds are, for example, Finquat CT-P from the company Finetex (Quaternium-89), Finquat CT from the company Finetex (Quaternium 75) and Condicate CT from the company Innospec Active Chemicals (Quaternium-75);
- quaternary ammonium salts containing at least one ester function, such as those of formula (XXIV) below: 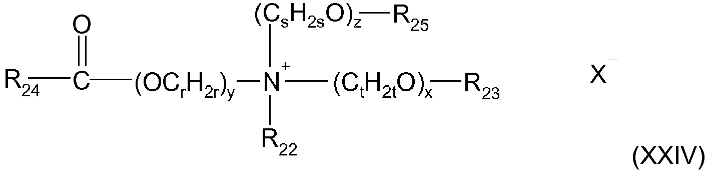 in which:
   R₂₂ is chosen from C₁-C₆ alkyl groups and C₁-C₆ hydroxyalkyl or dihydroxyalkyl groups;
   R₂₃ is chosen from:
      - the group 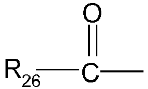
      - groups R₂₇ which are linear or branched, saturated or unsaturated C₁-C₂₂ hydrocarbon-based groups,
      - a hydrogen atom,
   R₂₅ is chosen from:
      - the group 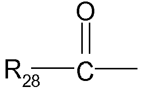
      - groups R₂₉ which are linear or branched, saturated or unsaturated C₁-C₆ hydrocarbon-based groups,
      - a hydrogen atom,
   R₂₄, R₂₆ and R₂₈, which may be identical or different, are chosen from linear or branched, saturated or unsaturated C₇-C₂₁ hydrocarbon-based groups;
   r, s and t, which may be identical or different, are integers ranging from 2 to 6;
   y is an integer ranging from 1 to 10;
   x and z, which may be identical or different, are integers ranging from 0 to 10;
   X⁻ is a simple or complex, organic or inorganic anion;
   with the proviso that the sum x + y + z is from 1 to 15, that when x is 0, then R₂₃ denotes R₂₇ and that when z is 0, then R₂₅ denotes R₂₉.

The alkyl groups R₂₂ may be linear or branched, and more particularly linear.

Preferably, R₂₂ denotes a methyl, ethyl, hydroxyethyl or dihydroxypropyl group, and more particularly a methyl or ethyl group.

Advantageously, the sum x + y + z is from 1 to 10.

When R₂₃ is a hydrocarbon-based group R₂₇, it may be long and contain from 12 to 22 carbon atoms, or may be short and contain from 1 to 3 carbon atoms.

When R₂₅ is a hydrocarbon-based group R₂₉, it preferably contains 1 to 3 carbon atoms.

Advantageously, R₂₄, R₂₆ and R₂₈, which may be identical or different, are chosen from linear or branched, saturated or unsaturated C₁₁-C₂₁ hydrocarbon-based groups, and more particularly from linear or branched, saturated or unsaturated C₁₁-C₂₁ alkyl and alkenyl groups.

Preferably, x and z, which may be identical or different, are equal to 0 or 1.

Advantageously, y is equal to 1.

Preferably, r, s and t, which may be identical or different, are equal to 2 or 3, and even more particularly are equal to 2.

The anion X⁻ is preferably a halide (chloride, bromide or iodide) or an alkyl sulfate, more particularly methyl sulfate. However, use may be made of methanesulfonate, phosphate, nitrate, tosylate, an anion derived from an organic acid, such as acetate or lactate, or any other anion compatible with the ammonium containing an ester function.

The anion X⁻ is even more particularly chloride or methyl sulfate.

Use is made more particularly, in the composition according to the invention, of the ammonium salts of formula (XXIV) in which:
R₂₂ denotes a methyl or ethyl group,
x and y are equal to 1;
z is equal to 0 or 1;
r, s and t are equal to 2;
R₂₃ is chosen from:
   - the group 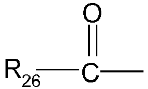
   - methyl, ethyl or C₁₄-C₂₂ hydrocarbon-based groups;
   - a hydrogen atom;
R₂₅ is chosen from:
   - the group 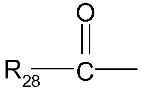
   - a hydrogen atom;

R₂₄, R₂₆ and R₂₈, which may be identical or different, are chosen from linear or branched, saturated or unsaturated C₁₃-C₁₇ hydrocarbon-based groups, and preferably from linear or branched, saturated or unsaturated C₁₃-C₁₇ alkyl and alkenyl groups.

The hydrocarbon-based groups are advantageously linear.

Mention may be made, for example, of the compounds of formula (XXIV) such as the diacyloxyethyldimethylammonium, diacyloxyethylhydroxyethylmethylammonium, monoacyloxyethyldihydroxyethylmethylammonium, triacyloxyethylmethylammonium and monoacyloxyethylhydroxyethyldimethylammonium salts (chloride or methyl sulfate in particular), and mixtures thereof. The acyl groups preferably contain 14 to 18 carbon atoms and are obtained more particularly from a plant oil such as palm oil or sunflower oil. When the compound contains several acyl groups, these groups may be identical or different.

These products are obtained, for example, by direct esterification of triethanolamine, of triisopropanolamine, of alkyldiethanolamine or of alkyldiisopropanolamine, which are optionally alkoxylated, with C₁₀-C₃₀ fatty acids or with mixtures of C₁₀-C₃₀ fatty acids of plant or animal origin, or by transesterification of their methyl esters. This esterification is followed by quaternization using an alkylating agent such as an alkyl (preferably methyl or ethyl) halide, a dialkyl (preferably methyl or ethyl) sulfate, methyl methanesulfonate, methyl para-toluenesulfonate, glycol chlorohydrin or glycerol chlorohydrin.

Such compounds are sold, for example, under the names Dehyquart® by the company Henkel, Stepanquat® by the company Stepan, Noxamium® by the company Ceca or Rewoquat® WE 18 by the company Rewo-Witco.

The composition according to the invention may contain, for example, a mixture of quaternary ammonium monoester, diester and triester salts with a weight majority of diester salts.

It is also possible to use the ammonium salts containing at least one ester function that are described in Patents US-A-4 874 554 and US-A-4 137 180.

The particularly preferred cationic surfactant(s) that may be used according to the invention are selected from compounds of formula (XXI) or of formula (XXIV), methyl(C₉-C₁₉)alkyl(C₁₀-C₂₀)alkylamidoethylimidazolium salts, and stearamidopropyldimethylamine.

Among all of the cationic surfactants that may be present in the composition according to the invention, it is preferred to select cetyltrimethylammonium, behenyltrimethylammonium, di(palmitoyloxyethyl)hydroxyethylmethylammonium, di(stearoyloxyethyl)hydroxyethylmethylammonium, methyl(C₉-C₁₉)alkyl(C₁₀-C₂₀)alkylamidoethylimidazolium salts, stearamidopropyltrimethylammonium salt, stearamidopropyldimethylamine, stearamidopropyldimethylcetearylammonium salt, and mixtures thereof.

When the conditioning agent of the composition according to the invention is a hydrocarbon, it is a linear or branched C₈-C₃₀ hydrocarbon.

Among the hydrocarbons that are liquid at room temperature corresponding to this definition, mention may be made especially of isododecane, isohexadecane and isomers thereof (such as 2,2,4,4,6,6-heptamethylnonane), isoeicosane, isotetracosane, isomers of the said compounds, n-nonadecane, n-dodecane, n-undecane, n-tridecane and n-pentadecane, and mixtures of these hydrocarbons.

Use is preferably made according to the invention of isododecane or an isomer thereof, or liquid petroleum jelly.

When the conditioning agent is a fatty alcohol, this alcohol is a linear or branched, saturated or unsaturated C₈-C₃₀ alcohol. Among the latter, mention may, for example, be made of 2-butyloctanol, lauryl alcohol, 2-octyldodecanol, oleyl alcohol, isocetyl alcohol, isostearyl alcohol, stearyl alcohol, cetyl alcohol and behenyl alcohol, and mixtures thereof.

The ceramides or ceramide analogues, such as glycoceramides, that may be used as conditioning agent in the compositions according to the invention are known *per se* and are natural or synthetic molecules that may correspond to the general formula (XXV) below: 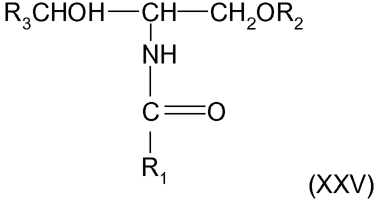 in which:
- R₁ denotes a linear or branched, saturated or unsaturated alkyl group, derived from C₁₄-C₃₀ fatty acids, it being possible for this group to be substituted with a hydroxyl group in the alpha position, or a hydroxyl group in the omega position esterified with a saturated or unsaturated C₁₆-C₃₀ fatty acid;
- R₂ denotes a hydrogen atom or a (glycosyl)ₙ, (galactosyl)ₘ or sulfogalactosyl group, in which n is an integer ranging from 1 to 4 and m is an integer ranging from 1 to 8;
- R₃ denotes a C₁₅-C₂₆ hydrocarbon-based group which is saturated or unsaturated in the alpha position, it being possible for this group to be substituted with one or more C₁-C₁₄ alkyl groups;

it being understood that, in the case of natural ceramides or glycoceramides, R₃ can also denote a C₁₅-C₂₆ α-hydroxyalkyl group, the hydroxyl group being optionally esterified with a C₁₆-C₃₀ α-hydroxy acid.

The ceramides which are preferred in the context of the present invention are those described by Downing in Arch. Dermatol., Vol. 123, 1381-1384, 1987, or those described in French patent FR 2 673 179.

The ceramide(s) that are more particularly preferred according to the invention are the compounds for which R₁ denotes a saturated or unsaturated alkyl derived from C₁₆-C₂₂ fatty acids; R₂ denotes a hydrogen atom; and R₃ denotes a saturated linear C₁₅ group.

Such compounds are, for example:
- N-linoleyldihydrosphingosine,
- N-oleyldihydrosphingosine,
- N-palmitoyldihydrosphingosine,
- N-stearoyldihydrosphingosine,
- N-behenyldihydrosphingosine,
or mixtures of these compounds.

Even more preferentially, ceramides are used for which R₁ denotes a saturated or unsaturated alkyl group derived from fatty acids; R₂ denotes a galactosyl or sulfogalactosyl group; and R₃ denotes a -CH=CH-(CH₂)₂-CH₃ group.

Among all these conditioning agents, use is preferably made of one or more conditioning agents selected from cationic polymers.

The composition, especially a cosmetic composition, according to the invention preferably contains from 0.01% to 20% by weight, and more preferentially from 0.05% to 10% by weight of conditioning agent(s), relative to the total weight of the composition.

The composition according to the invention may also comprise one or more standard additives that are well known in the art, other than the compounds defined previously. As examples of additives that may be used according to the invention, mention may be made of anionic surfactants, amphoteric or zwitterionic surfactants, nonionic surfactants, proteins or protein hydrolysates, vitamins, reducing agents, plasticizers, softeners, antifoams, moisturizers, pigments, clays, mineral fillers, UV-screening agents, abrasive agents (pumice, apricot kernel powder), mineral colloids, peptizers, solubilizers, fragrances, preservatives, nacreous agents, propellants, antidandruff agents (for example, zinc pyrithione, octopirox, selenium sulfide, ellagic acid and derivatives), agents for combating hair loss or agents for promoting hair regrowth; these additives being other than the compounds defined above.

A person skilled in the art will take care to select the optional additive(s) and the amount thereof such that they do not harm the properties of the compositions of the present invention.

The additive(s) are generally present in the composition according to the invention in an amount ranging from 0 to 20% by weight relative to the total weight of the composition.

The compositions according to the invention may be provided in all the formulation forms conventionally used for a topical application and in particular in the form of aqueous or aqueous/alcoholic solutions, of oil-in-water (O/W), water-in-oil (W/O) or multiple (triple: W/O/W or O/W/O) emulsions, of aqueous gels or of dispersions of a fatty phase in an aqueous phase using spherules, it being possible for these spherules to be polymeric nanoparticles, such as nanospheres and nanocapsules, or lipid vesicles of ionic and/or nonionic type (liposomes, niosomes or oleosomes). These compositions are prepared according to the usual methods.

In addition, the compositions used according to the invention can be more or less fluid and can have the appearance of a white or coloured cream, of an ointment, of a milk, of a lotion, of a serum, of a paste or of a foam. They can optionally be applied to the keratin materials in aerosol form. They can also be in solid form, for example in the form of a stick.

Of course, the person skilled in the art will take care to choose the optional compound or compounds to be added to the composition according to the invention so that the advantageous properties intrinsically attached to the composition in accordance with the invention are not, or not substantially, adversely affected by the envisaged addition or additions.

As indicated above, a further subject of the invention is a method for the nontherapeutic cosmetic treatment of keratin materials, in particular the hair, comprising the application, to said materials, of a composition as described above.

This application may or may not be followed by rinsing.

When the application of the composition is followed by a rinsing operation, the leave-in time of the composition on the keratin materials ranges from a few seconds to 60 minutes, better still from 5 seconds to 30 minutes, even better still from 10 seconds to 10 minutes.

Whether in rinsed mode or non-rinsed mode, the application of the composition may take place in the presence of heat. The heating device may be a hairdryer, a hood dryer, a curling iron or a flat iron. The heating temperature may be between 40°C and 220°C.

The application of the composition according to the invention to the hair may take place on dry hair or on wet hair. It may in particular be carried out after a shampooing operation or after a pretreatment at acidic or basic pH.

A subject of the invention is also the use of a composition according to the invention for caring for and/or shaping keratin materials, especially keratin fibres and in particular human keratin fibres such as the hair.

The examples that follow are intended to illustrate the invention without however, being limiting in nature.

### Example 1:

Compositions (A1) and (A2) are prepared from the ingredients indicated in the table below, the amounts of which are expressed as weight percentages of active material relative to the total weight of the composition.

| Compositions | A1 | A2 |
|---|---|---|
| 3-Aminopropyltriethoxysilane | 5 | 5 |
| Hydroxypropyl corn starch phosphate (Structure ZEA from Akzo Nobel) | 4.4 | - |
| Hydroxypropyl guar (Jaguar HP 105 from Rhodia) | - | 2 |
| Hydroxyethylcellulose (Natrosol 250 HHR from Ashland) | 0.7 | 0.7 |
| Lactic acid | 1.75 | 1.75 |
| pH agent qs | pH 9 | pH 9 |
| Deionized water | qs 100% | qs 100% |

Composition B1 is prepared from the ingredients indicated in the table below, the amounts of which are expressed as weight percentages of active material relative to the total weight of the composition.

| Composition | B1 |
|---|---|
| Bis(diglyceryl) poly(2-acyladipate) (Softisan 649 from Sasol) | 0.15 |
| Amino silicone in an aqueous emulsion containing 60% AM (Dow Corning 2-8299 Cationic Emulsion from Dow Corning) | 0.96 |
| Hydroxyethylcellulose (Natrosol 250 HHR from Ashland) | 0.2 |
| Cetylstearyl alcohol (C16/C18 50/50 by weight) | 3 |
| Mixture of myristyl myristate, cetyl palmitate and stearyl stearate (Miraceti from Laserson) | 0.25 |
| Lauryl PEG/PPG-18/18 methicone at 72% active material (Dow Corning 5200 Formulation Aid - Dow Corning) | 0.18 |
| Behenyltrimethylammonium chloride as a 79% solution in isopropanol (Genamin KDMP from Clariant) | 1.58 |
| pH agent qs | pH 3.5 |
| PEG-180 (Polyglycol 8000 S from Clariant) | 2 |
| L-Glycine | 0.01 |
| D-Panthenol | 0.1 |
| Vitamin E acetate | 0.1 |
| 2-Oleamido-1,3-octadecanediol | 0.01 |
| Fragrance | qs |
| Preserving agents, dyes | qs |
| Water | qs 100% |

Compositions (A1) and (B1) or (A2) and (B1) are conditioned in a two-bodied device consisting of two compartments and of a dispensing head for dispensing a mixture consisting of equal amounts of compositions A1 and B1 or A2 and B1.

When applied as a rinse-out conditioner, the mixture of compositions (A1) and (B1) or (A2) and (B1) (50/50 by weight) gives the hair body, volume and also a uniform feel from the root to the end.

Furthermore, this composition makes it possible to facilitate the shaping of fine hair and to give a better curl definition to curly hair.

### Example 3: Leave-in care product

The leave-in care composition (3) is prepared from the ingredients indicated in the table below, the amounts of which are expressed as weight percentages of active material relative to the total weight of the composition.

| Composition | 3 (invention) |
|---|---|
| 3-Aminopropyltrimethoxysilane (KBE-903 from Shin-Etsu) | 2 |
| Amino silicone in an aqueous emulsion containing 60% AM (Dow Corning 2-8299 Cationic Emulsion from Dow Corning) | 0.9 |
| cetylstearyl alcohol (C16/C18 50/50) | 5 |
| Mixture of myristyl myristate, cetyl palmitate and stearyl stearate (Miraceti from Laserson) | 1 |
| Behenyltrimethylammonium chloride as a 79% solution in isopropanol (Genamin KDMP from Clariant) | 0.8 |
| Lactic acid qs | pH 9 |
| Fragrance, preservatives qs | |
| Water | qs 100% |

## Claims

1. Composition, especially a cosmetic composition, comprising:
(i) one or more alkoxysilanes,
(ii) one or more fatty esters; and
(iii) one or more silicones.

2. Composition according to the preceding claim, **characterized in that** the alkoxysilane is chosen from the following compounds:
- compounds of formula (I) : 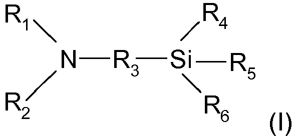 in which:
R₄ represents a halogen or a group ORₐ or R₁ₐ;
R₅ represents a halogen or a group OR_{b} or R₂ₐ;
R₆ represents a halogen or a group OR_{c} or R₃ₐ;
R₁ and R₂ represent, independently of each other, a hydrogen atom or a saturated or unsaturated, linear or branched hydrocarbon-based group, preferably R₁ or R₂ necessarily denoting a hydrogen atom,
R₃, Rₐ, R_{b}, R_{c}, R₁ₐ, R₂ₐ and R₃ₐ represent, independently of each other, a saturated or unsaturated, linear or branched hydrocarbon-based group, optionally bearing additional chemical groups such as acid or amine groups, Rₐ, R_{b} and R_{c} also possibly denoting hydrogen, and at least two of the groups R₄, R₅ and R₆ being other than groups R₁ₐ, R₂ₐ and R₃ₐ, and preferably at least two of the groups Rₐ, R_{b} and R_{c} being other than hydrogen.
- compounds of formula (II):
(R₂₁O)x(R₂₂)ySi-(B)p-[NR₂₃-(Ba)pa]q-[NR₂₃ₐ-(B_{b})pb]qa-Si(R₂₂ₐ)ya(OR₂₁ₐ)xa
in which:
R₂₁, R₂₂, R₂₁ₐ and R₂₂ₐ represent, independently of each other, a saturated or unsaturated, linear or branched hydrocarbon-based chain, optionally containing one or more heteroatoms, optionally interrupted or substituted with one or more groups chosen from ether, ester, amine, amide, carboxyl, hydroxyl and carbonyl groups,
R₂₃ and R₂₃ₐ each independently represent a hydrogen atom or a saturated or unsaturated, linear or branched hydrocarbon-based chain, optionally containing one or more heteroatoms, optionally interrupted or substituted with one or more ether, C₁-C₂₀ alcohol ester, amine, carboxyl, C₆-C₃₀ aryl, hydroxyl or carbonyl groups, or a heterocyclic or non-heterocyclic aromatic ring, optionally substituted with one or more C₁-C₂₀ alcohol ester, amine, amide, carboxyl, hydroxyl, carbonyl or acyl groups, R₂₃ and R₂₃ₐ preferably representing a hydrogen atom,
x is an integer ranging from 1 to 3, y = 3-x, xa is an integer ranging from 1 to 3, ya = 3-xa, p = 0 or 1, pa = 0 or 1, pb = 0 or 1, q = 0 or 1, qa = 0 or 1, it being understood that at least q or qa is other than zero,
B, Bₐ and B_{b} each independently represent a linear or branched divalent C₁-C₂₀ alkylene radical.
- compounds of formula (III): 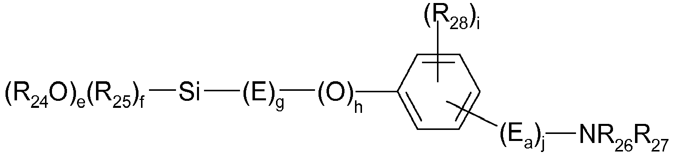 in which:
R₂₄ and R₂₅ represent, independently of each other, a saturated or unsaturated, linear or branched hydrocarbon-based chain, optionally containing one or more heteroatoms, optionally interrupted or substituted with one or more groups chosen from ether, ester, amine, amide, carboxyl, hydroxyl and carbonyl groups,
e = 2 or 3;
f = 3-e;
g = 0 or 1;
j = 0 or 1;
E and Eₐ each independently represent a linear or branched divalent C₁-C₂₀ alkylene radical,
R₂₆ and R₂₇ each independently represent a hydrogen atom or a saturated or unsaturated, linear or branched hydrocarbon-based chain, optionally containing one or more heteroatoms, optionally interrupted or substituted with one or more ether, C₁-C₂₀ alcohol ester, amine, carboxyl, C₆-C₃₀ aryl, hydroxyl or carbonyl groups, or a heterocyclic or non-heterocyclic aromatic ring, optionally substituted with one or more C₁-C₂₀ alcohol ester, amine, amide, carboxyl, hydroxyl, carbonyl or acyl groups, R₂₆ or R₂₇ preferably representing a hydrogen atom,
i is an integer ranging from 0 to 4,
h is 0 or 1,
the group(s) R₂₈ each independently represent a hydrogen atom or a saturated or unsaturated, linear or branched, preferably C₁-C₁₀ hydrocarbon-based chain, optionally containing one or more heteroatoms, optionally interrupted or substituted with one or more ether, C₁-C₂₀ alcohol ester, amine, carboxyl, C₆-C₃₀ aryl, hydroxyl or carbonyl groups, or a heterocyclic or non-heterocyclic aromatic ring, optionally substituted with one or more C₁-C₂₀ alcohol ester, amine, amide, carboxyl, hydroxyl, carbonyl or acyl groups.

3. Composition according to either of the preceding claims, **characterized in that** the alkoxysilane is of formula (I) below: 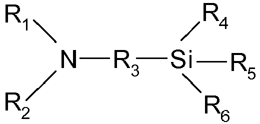 in which:
R₁ and R₂, independently of each other, are chosen from hydrogen and ethyl, propyl and aminoethyl groups;
R₃ is chosen from ethyl, propyl and methylphenethyl groups;
R₄, R₅ and R₆, independently of each other, are chosen from methyl, methoxy and ethoxy groups.

4. Composition according to any one of the preceding claims, **characterized in that** the alkoxysilane is chosen from alkoxysilanes comprising one or more amine functions, which are preferably primary and/or secondary.

5. Composition according to any one of the preceding claims, **characterized in that** the alkoxysilane is chosen from 3-aminopropyltriethoxysilane, 3-aminopropylmethyldiethoxysilane, N-(2-aminoethyl)-3-aminopropyltriethoxysilane and 3-(2-aminoethylamino)propylmethyldiethoxysilane, and is preferably 3-aminopropyltriethoxysilane.

6. Composition according to any one of the preceding claims, **characterized in that** the alkoxysilane is present in the composition in an amount ranging from 0.05% to 20% by weight, in particular from 0.1% to 10% and preferably from 0.2% to 5% by weight relative to the total weight of the composition.

7. Composition according to any one of the preceding claims, **characterized in that** the fatty esters are chosen from:
- esters of saturated, linear or branched C₁-C₃₀ monoalcohols, with C₁₀-C₃₀ monofunctional fatty acids, the latter possibly being linear or branched, and saturated or unsaturated;
- esters of linear or branched C₃-C₈ monoalcohols, with C₁₀-C₃₀ difunctional fatty acids, the latter possibly being linear or branched, and saturated or unsaturated;
- esters and diesters of sugars and of C₁₀-C₃₀ fatty acids;
- mixtures thereof.

8. Composition according to any one of the preceding claims, **characterized in that** the fatty esters are solid at 25°C and at atmospheric pressure (10⁵ Pa).

9. Composition according to any one of the preceding claims, **characterized in that** the fatty esters are chosen from myristyl myristate, cetyl myristate, stearyl myristate, myristyl palmitate, cetyl palmitate, stearyl palmitate, myristyl stearate, cetyl stearate, stearyl stearate and behenyl behenate, and mixtures thereof.

10. Composition according to any one of Claims 1 to 7, **characterized in that** the fatty esters are liquid at 25°C and at atmospheric pressure (10⁵ Pa).

11. Composition according to any one of Claims 1 to 7 and 10, **characterized in that** the fatty esters are chosen from isopropyl palmitate, isopropyl myristate, octyldodecyl stearate and isononyl isononanoate, and mixtures thereof.

12. Composition according to any one of the preceding claims, **characterized in that** the fatty ester(s) represent from 0.01% to 10% by weight and preferably from 0.1% to 5% by weight relative to the total weight of the composition.

13. Composition according to any one of the preceding claims, **characterized in that** the silicones are chosen from polyalkylsiloxanes, polyarylsiloxanes, polyalkylarylsiloxanes, silicone gums and resins, and polyorganosiloxanes modified with organofunctional groups, and also mixtures thereof.

14. Composition according to any one of the preceding claims, **characterized in that** the silicones are chosen from polydialkylsiloxanes bearing trimethylsilyl end groups, polydialkylsiloxanes bearing dimethylsilanol end groups and polysiloxanes bearing amino groups.

15. Composition according to any one of the preceding claims, in which the silicone(s) represent(s) from 0.01% to 10% by weight, preferably from 0.3% to 5% by weight and more particularly from 0.3% to 3% by weight relative to the total weight of the composition.

16. Process for the non-therapeutic cosmetic treatment of keratin materials, especially keratin fibres and in particular human keratin fibres such as the hair, comprising the application, to the said materials, of a composition as described in any one of Claims 1 to 15, the composition being rinsed out or left in after application.

17. Use of a composition as defined according to one of Claims 1 to 15, for caring for and/or shaping keratin materials, especially keratin fibres and in particular human keratin fibres such as the hair.

## Patentansprüche

1. Zusammensetzung, insbesondere kosmetische Zusammensetzung, umfassend:
(i) ein oder mehrere Alkoxysilane,
(ii) einen oder mehrere Fettsäureester und
(iii) ein oder mehrere Silikone.

2. Zusammensetzung nach dem vorhergehenden Anspruch, **dadurch gekennzeichnet, dass** das Alkoxysilan aus den folgenden Verbindungen ausgewählt ist:
- Verbindungen der Formel (I): 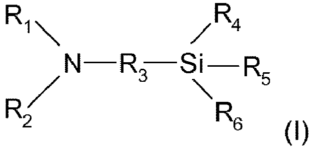 worin:
R₄ für ein Halogen oder eine Gruppe ORₐ oder R₁ₐ steht;
R₅ für ein Halogen oder eine Gruppe OR_{b} oder R₂ₐ steht;
R₆ für ein Halogen oder eine Gruppe OR_{c} oder R₃ₐ steht;
R₁ und R₂ unabhängig voneinander für ein Wasserstoffatom oder eine gesättigte oder ungesättigte, lineare oder verzweigte kohlenwasserstoffbasierte Gruppe stehen, wobei vorzugsweise R₁ oder R₂ zwingend für ein Wasserstoffatom stehen,
R₃, Rₐ, R_{b}, R_{c}, R₁ₐ, R₂ₐ und R₃ₐ unabhängig voneinander für eine gesättigte oder ungesättigte, lineare oder verzweigte kohlenwasserstoffbasierte Gruppe, die gegebenenfalls zusätzliche chemische Gruppen wie Säure- oder Amingruppen trägt, stehen, wobei Rₐ, R_{b} und R_{c} außerdem Wasserstoff bedeuten können und mindestens zwei der Gruppen R₄, R₅ und R₆ von den Gruppen R₁ₐ, R₂ₐ und R₃ₐ verschieden sind und vorzugsweise mindestens zwei der Gruppen Rₐ, R_{b} und R_{c} von Wasserstoff verschieden sind;
- Verbindungen der Formel (II):
(R₂₁O)ₓ(R₂₂)_{y}Si-(B)ₚ-[NR₂₃-(Bₐ)ₚₐ]_{q}-[NR₂₃ₐ-(B_{b})_{pb}]_{qa}-Si(R₂₂ₐ)_{ya}(OR₂₁ₐ)ₓₐ
worin:
R₂₁, R₂₂, R₂₁ₐ und R₂₂ₐ unabhängig voneinander für eine gesättigte oder ungesättigte, lineare oder verzweigte kohlenwasserstoffbasierte Kette, die gegebenenfalls ein oder mehrere Heteroatome enthält und gegebenenfalls durch eine oder mehrere Gruppen, die aus Ether-, Ester-, Amin-, Amid-, Carboxyl-, Hydroxyl- und Carbonylgruppen ausgewählt sind, unterbrochen oder substituiert ist, stehen,
R₂₃ und R₂₃ₐ jeweils unabhängig für ein Wasserstoffatom oder eine gesättigte oder ungesättigte, lineare oder verzweigte kohlenwasserstoffbasierte Kette, die gegebenenfalls ein oder mehrere Heteroatome enthält und gegebenenfalls durch eine oder mehrere Ether-, C₁-C₂₀-Alkoholester-, Amin-, Carboxyl-, C₆-C₃₀-Aryl-, Hydroxyl- oder Carbonylgruppen unterbrochen oder substituiert ist, oder einen heterocyclischen oder nicht heterocyclischen aromatischen Ring, der gegebenenfalls durch eine oder mehrere C₁-C₂₀-Alkoholester-, Amin-, Amid-, Carboxyl-, Hydroxyl-, Carbonyl- oder Acylgruppen substituiert ist, stehen, wobei R₂₃ und R₂₃ₐ vorzugsweise für ein Wasserstoffatom stehen,
x für eine ganze Zahl im Bereich von 1 bis 3 steht, y = 3-x, xa für eine ganze Zahl im Bereich von 1 bis 3 steht, ya = 3-xa, p = 0 oder 1, pa = 0 oder 1, pb = 0 oder 1, q = 0 oder 1, qa = 0 oder 1, mit der Maßgabe, dass mindestens q oder qa von null verschieden ist,
B, Bₐ und B_{b} jeweils unabhängig für einen linearen oder verzweigten zweiwertigen C₁-C₂₀-Alkylenrest stehen,
- Verbindungen der Formel (III): 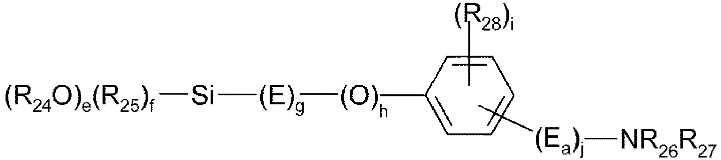 worin:
R₂₄ und R₂₅ unabhängig voneinander für eine gesättigte oder ungesättigte, lineare oder verzweigte kohlenwasserstoffbasierte Kette, die gegebenenfalls ein oder mehrere Heteroatome enthält und gegebenenfalls durch eine oder mehrere Gruppen, die aus Ether-, Ester-, Amin-, Amid-, Carboxyl-, Hydroxyl- und Carbonylgruppen ausgewählt sind, unterbrochen oder substituiert ist, stehen,
e = 2 oder 3;
f = 3-e;
g = 0 oder 1;
j = 0 oder 1;
E und Eₐ jeweils unabhängig für einen linearen oder verzweigten zweiwertigen C₁-C₂₀-Alkylenrest stehen, R₂₆ und R₂₇ jeweils unabhängig für ein Wasserstoffatom oder eine gesättigte oder ungesättigte, lineare oder verzweigte kohlenwasserstoffbasierte Kette, die gegebenenfalls ein oder mehrere Heteroatome enthält und gegebenenfalls durch eine oder mehrere Ether-, C₁-C₂₀-Alkoholester-, Amin-, Carboxyl-, C₆-C₃₀-Aryl-, Hydroxyl- oder Carbonylgruppen unterbrochen oder substituiert ist, oder einen heterocyclischen oder nicht heterocyclischen aromatischen Ring, der gegebenenfalls durch eine oder mehrere C₁-C₂₀-Alkoholester-, Amin-, Amid-, Carboxyl-, Hydroxyl-, Carbonyl- oder Acylgruppen substituiert ist, stehen, wobei R₂₆ oder R₂₇ vorzugsweise für ein Wasserstoffatom steht,
i für eine ganze Zahl im Bereich von 0 bis 4 steht,
h für 0 oder 1 steht,
die Gruppe bzw. Gruppen R₂₈ jeweils unabhängig für ein Wasserstoffatom oder eine gesättigte oder ungesättigte, lineare oder verzweigte kohlenwasserstoffbasierte Kette, vorzugsweise C₁-C₁₀-kohlenwasserstoffbasierte Kette, die gegebenenfalls ein oder mehrere Heteroatome enthält und gegebenenfalls durch eine oder mehrere Ether-, C₁-C₂₀-Alkoholester-, Amin-, Carboxyl-, C₆-C₃₀-Aryl-, Hydroxyl- oder Carbonylgruppen unterbrochen oder substituiert ist, oder einen heterocyclischen oder nicht heterocyclischen aromatischen Ring, der gegebenenfalls durch eine oder mehrere C₁-C₂₀-Alkoholester-, Amin-, Amid-, Carboxyl-, Hydroxyl-, Carbonyl- oder Acylgruppen substituiert ist, stehen.

3. Zusammensetzung nach einem der vorhergehenden Ansprüche, **dadurch gekennzeichnet, dass** das Alkoxysilan die nachstehende Formel (I) aufweist: 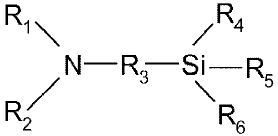 worin:
R₁ und R₂ unabhängig voneinander aus Wasserstoff und Ethyl-, Propyl- und Aminoethylgruppen ausgewählt sind,
R₃ aus Ethyl-, Propyl- und Methylphenethylgruppen ausgewählt ist,
R₄, R₅ und R₆ unabhängig voneinander aus Methyl-, Methoxy- und Ethoxygruppen ausgewählt sind.

4. Zusammensetzung nach einem der vorhergehenden Ansprüche, **dadurch gekennzeichnet, dass** das Alkoxysilan aus Alkoxysilanen mit einer oder mehreren Aminfunktionen, die vorzugsweise primär und/oder sekundär sind, ausgewählt ist.

5. Zusammensetzung nach einem der vorhergehenden Ansprüche, **dadurch gekennzeichnet, dass** das Alkoxysilan aus 3-Aminopropyltriethoxysilan, 3-Aminopropylmethyldiethoxysilan, N-(2-Aminoethyl)-3-aminopropyltriethoxysilan und 3-(2-Aminoethyl-amino)propylmethyldiethoxysilan ausgewählt ist und vorzugsweise 3-Aminopropyltriethoxysilan ist.

6. Zusammensetzung nach einem der vorhergehenden Ansprüche, **dadurch gekennzeichnet, dass** das Alkoxysilan in der Zusammensetzung in einer Menge im Bereich von 0,05 bis 20 Gew.-%, insbesondere von 0,1 bis 10 Gew.-% und vorzugsweise von 0,2 bis 5 Gew.-%, bezogen auf das Gesamtgewicht der Zusammensetzung, vorliegt.

7. Zusammensetzung nach einem der vorhergehenden Ansprüche, **dadurch gekennzeichnet, dass** die Fettsäureester aus:
- Estern von gesättigten, linearen oder verzweigten C₁-C₃₀-Monoalkoholen mit monofunktionellen C₁₀-C₃₀-Fettsäuren, wobei letztere linear oder verzweigt und gesättigt oder ungesättigt sein können;
- Estern von linearen oder verzweigten C₃-C₈-Monoalkoholen mit difunktionellen C₁₀-C₃₀-Fettsäuren, wobei letztere linear oder verzweigt und gesättigt oder ungesättigt sein können;
- Estern und Diestern von Zuckern und C₁₀-C₃₀-Fettsäuren;
- Mischungen davon
ausgewählt sind.

8. Zusammensetzung nach einem der vorhergehenden Ansprüche, **dadurch gekennzeichnet, dass** die Fettsäureester bei 25°C und bei Normaldruck (10⁵ Pa) fest sind.

9. Zusammensetzung nach einem der vorhergehenden Ansprüche, **dadurch gekennzeichnet, dass** die Fettsäureester aus Myristylmyristat, Cetylmyristat, Stearylmyristat, Myristylpalmitat, Cetylpalmitat, Stearylpalmitat, Myristylstearat, Cetylstearat, Stearylstearat und Behenylbehenat und Mischungen davon ausgewählt sind.

10. Zusammensetzung nach einem der Ansprüche 1 bis 7, **dadurch gekennzeichnet, dass** die Fettsäureester bei 25°C und bei Normaldruck (10⁵ Pa) flüssig sind.

11. Zusammensetzung nach einem der Ansprüche 1 bis 7 und 10, **dadurch gekennzeichnet, dass** die Fettsäureester aus Isopropylpalmitat, Isopropylmyristat, Octyldodecylstearat und Isononylisononanoat und Mischungen davon ausgewählt sind.

12. Zusammensetzung nach einem der vorhergehenden Ansprüche, **dadurch gekennzeichnet, dass** der Fettsäureester bzw. die Fettsäureester 0,01 bis 10 Gew.-% und vorzugsweise 0,1 bis 5 Gew.-%, bezogen auf das Gesamtgewicht der Zusammensetzung, ausmacht bzw. ausmachen.

13. Zusammensetzung nach einem der vorhergehenden Ansprüche, **dadurch gekennzeichnet, dass** die Silikone aus Polyalkylsiloxanen, Polyarylsiloxanen, Polyalkylarylsiloxanen, Silikongummen und -harzen und mit organofunktionellen Gruppen modifizierten Polyorganosiloxanen sowie Mischungen davon ausgewählt sind.

14. Zusammensetzung nach einem der vorhergehenden Ansprüche, **dadurch gekennzeichnet, dass** die Silikone aus Polydialkylsiloxanen mit Trimethylsilyl-Endgruppen, Polydialkylsiloxanen mit Dimethylsilanol-Endgruppen und Polysiloxanen mit Aminogruppen ausgewählt sind.

15. Zusammensetzung nach einem der vorhergehenden Ansprüche, wobei das Silikon bzw. die Silikone 0,01 bis 10 Gew.-%, vorzugsweise 0,3 bis 5 Gew.-% und spezieller 0,3 bis 3 Gew.-%, bezogen auf das Gesamtgewicht der Zusammensetzung, ausmacht bzw. ausmachen.

16. Verfahren zur nichttherapeutischen kosmetischen Behandlung von Keratinmaterialien, speziell Keratinfasern und insbesondere menschlichen Keratinfasern wie dem Haar, bei dem man auf die Materialien eine Zusammensetzung gemäß einem der Ansprüche 1 bis 15 aufbringt, wobei die Zusammensetzung nach dem Aufbringen ausgespült oder in dem Material belassen wird.

17. Verwendung einer Zusammensetzung gemäß einem der Ansprüche 1 bis 15 zur Pflege und/oder Gestaltung von Keratinmaterialien, speziell Keratinfasern und insbesondere menschlichen Keratinfasern wie dem Haar.

## Revendications

1. Composition notamment cosmétique comprenant :
(i) un ou plusieurs alcoxysilanes,
(ii) un ou plusieurs esters gras, et
(iii) une ou plusieurs silicones.

2. Composition selon la revendication précédente, **caractérisée en ce que** l'alcoxysilane est choisi parmi les composés suivants :
- composés de formule (I) : 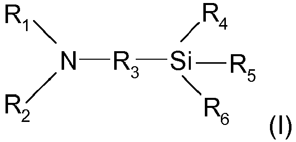 dans laquelle :
R₄ représente un halogène, un groupe ORₐ ou R₁ₐ ;
R₅ représente un halogène, un groupe OR_{b} ou R₂ₐ ;
R₆ représente un halogène, un groupe OR_{c} ou R₃ₐ ;
R₁ et R₂, représentent, indépendamment l'un de l'autre, un atome d'hydrogène, un groupe hydrocarboné saturé ou insaturé, linéaire ou ramifié, de préférence R₁ ou R₂ désignant nécessairement un atome d'hydrogène,
R₃, Rₐ, R_{b}, R_{c}, R₁ₐ, R₂ₐ, R₃ₐ représentent, indépendamment les uns des autres, un groupe hydrocarboné saturé ou insaturé, linéaire ou ramifié, portant éventuellement des groupes chimiques supplémentaires tels que des groupes acides ou amines, Rₐ, R_{b} et R_{c} pouvant en outre désigner l'hydrogène, et deux au moins des groupes R₄, R₅ et R₆ étant différents des groupes R₁ₐ, R₂ₐ et R₃ₐ, de préférence deux au moins des groupes Rₐ, R_{b}, R_{c} étant différents de l'hydrogène.
- composés de formule (II) :
(R₂₁O)x(R₂₂)ySi-(B)p-[NR₂₃-(Bₐ)pa]q-[NR₂₃ₐ-(B_{b})pb]qa-Si(R₂₂ₐ)ya(OR₂₁ₐ)xa
dans laquelle :
R₂₁, R₂₂, R₂₁ₐ et R₂₂ₐ représentent chacun indépendamment, l'un de l'autre, une chaîne hydrocarbonée saturée ou insaturée, linéaire ou ramifiée, contenant éventuellement un ou plusieurs hétéroatomes, éventuellement interrompue ou substituée par un ou plusieurs groupes choisis parmi les groupes éther, ester, amine, amide, carboxyle, hydroxyle et carbonyle,
R₂₃ et R₂₃ₐ représentent chacun indépendamment un atome d'hydrogène ou une chaîne hydrocarbonée saturée ou insaturée, linéaire ou ramifiée, contenant éventuellement un ou plusieurs hétéroatomes, éventuellement interrompue ou substituée par un ou plusieurs groupes éther, ester d'alcool en C₁C₂₀, amine, carboxyle, aryle en C₆C₃₀, hydroxyle ou carbonyle, ou un cycle aromatique, hétérocyclique ou non, éventuellement substitué par un ou plusieurs groupes ester d'alcool en C₁C₂₀, amine, amide, carboxyle, hydroxyle, carbonyle ou acyle, de préférence, R₂₃ et R₂₃ₐ représentant un atome d'hydrogène,
x est un entier variant de 1 à 3, y = 3-x, xa est un entier variant de 1 à 3, ya = 3-xa, p = 0 ou 1, pa = 0 ou 1, pb = 0 ou 1, q = 0 ou 1, qa = 0 ou 1, étant entendu que au moins q ou qa est différent de zéro,
B, Bₐ et B_{b} représentent chacun indépendamment un radical divalent alkylène linéaire ou ramifié en C₁-C₂₀.
- les composés de formule (III) : 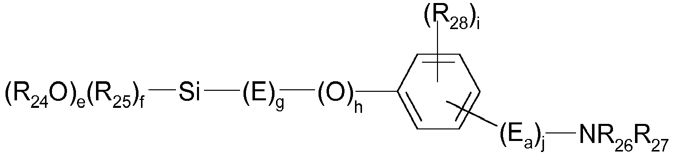 dans laquelle :
R₂₄ et R₂₅ représentent chacun indépendamment l'un de l'autre une chaîne hydrocarbonée saturée ou insaturée, linéaire ou ramifiée, contenant éventuellement un ou plusieurs hétéroatomes, éventuellement interrompue ou substituée par un ou plusieurs groupes choisis parmi les groupes éther, ester, amine, amide, carboxyle, hydroxyle et carbonyle,
e = 2 ou 3 ;
f = 3-e ;
g = 0 ou 1 ;
j = 0 ou 1 ;
E et Eₐ représentent chacun indépendamment un radical divalent alkylène linéaire ou ramifié en C₁-C₂₀,
R₂₆ et R₂₇ représentent chacun indépendamment l'hydrogène ou une chaîne hydrocarbonée saturée ou insaturée, linéaire ou ramifiée, contenant éventuellement un ou plusieurs hétéroatomes, éventuellement interrompue ou substituée par un ou plusieurs groupes éther, ester d'alcool en C₁-C₂₀, amine, carboxyle, aryle en C₆-C₃₀, hydroxyle ou carbonyle, ou un cycle aromatique, hétérocyclique ou non, éventuellement substitué par un ou plusieurs groupes ester d'alcool en C₁-C₂₀, amine, amide, carboxyle, hydroxyle, carbonyle ou acyle, R₂₆ ou R₂₇ désignant de préférence un atome d'hydrogène,
i est un entier variant de 0 à 4,
h vaut 0 ou 1,
le ou les groupes R₂₈ représentent chacun indépendamment l'hydrogène ou une chaîne hydrocarbonée saturée ou insaturée, linéaire ou ramifiée, de préférence en C₁-C₁₀, contenant éventuellement un ou plusieurs hétéroatomes, éventuellement interrompue ou substituée par un ou plusieurs groupes éther, ester d'alcool en C₁-C₂₀, amine, carboxyle, aryle en C₆-C₃₀, hydroxyle ou carbonyle, ou un cycle aromatique, hétérocyclique ou non, éventuellement substitué par un ou plusieurs groupes ester d'alcool en C₁-C₂₀, amine, amide, carboxyle, hydroxyle, carbonyle ou acyle.

3. Composition selon l'une quelconque des revendications précédentes, **caractérisée en ce que** l'alcoxysilane est de formule (I) suivante : 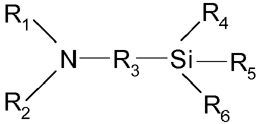 dans laquelle :
R₁ et R₂, indépendamment l'un de l'autre sont choisis parmi l'hydrogène et les groupes éthyle, propyle et aminoéthyle ;
R₃ est choisi parmi les groupes éthyle, propyle et méthylphénéthyle ;
R₄, R₅ et R₆ indépendamment l'un de l'autre sont choisis parmi les groupes méthyle, méthoxy, éthoxy.

4. Composition selon l'une quelconque des revendications précédentes, **caractérisée en ce que** l'alcoxysilane est choisi parmi les alcoxysilanes comportant une ou plusieurs fonctions amines, de préférence primaires et/ou secondaires.

5. Composition selon l'une quelconque des revendications précédentes, **caractérisée en ce que** l'alcoxysilane est choisi parmi le 3-aminopropyltriéthoxysilane, le 3-aminopropylméthyldiéthoxysilane, le N-(2-aminoéthyl)-3-aminopropyltriéthoxysilane, et le 3-(2-aminoéthylamino)propylméthyldiéthoxysilane et de préférence est le 3-aminopropyltriéthoxysilane.

6. Composition selon l'une quelconque des revendications précédentes, **caractérisée en ce que** l'alcoxysilane est présent dans la composition en une quantité allant de 0,05 % à 20 %, en particulier de 0,1 à 10 %, de préférence de 0,2 à 5 %, par rapport au poids total de la composition.

7. Composition selon l'une quelconque des revendications précédentes, **caractérisée par le fait que** les esters gras sont choisis parmi
- les esters de monoalcools saturés linéaires ou ramifiés en C₁-C₃₀, avec des acides gras monofonctionnels en C₁₀-C₃₀, ces derniers pouvant être linéaires ou ramifiés, saturés ou insaturés ;
- les esters de monoalcools linéaires ou ramifiés en C₃-C₈, avec des acides gras bifonctionnels en C₁₀-C₃₀, ces derniers pouvant être linéaires ou ramifiés, saturés ou insaturés ;
- les esters et di-esters de sucres et d'acides gras en C₁₀-C₃₀ ,
- leurs mélanges.

8. Composition selon l'une quelconque des revendications précédentes, **caractérisée par le fait que** les esters gras sont solides à 25°C et à la pression atmosphérique (10⁵ Pa).

9. Composition selon l'une quelconque des revendications précédentes **caractérisée par le fait que** les esters gras sont choisis parmi les myristates de myristyle, de cétyle et de stéaryle, les palmitates de myristyle, de cétyle et de stéaryle, les stéarates de myristyle, de cétyle et de stéaryle, le béhénate de béhényle et leurs mélanges.

10. Composition selon l'une quelconque des revendications 1 à 7, **caractérisée par le fait que** les esters gras sont liquides à 25°C et à la pression atmosphérique (10⁵ Pa).

11. Composition selon l'une quelconque des revendications 1 à 7 et 10, **caractérisée par le fait que** les esters gras sont choisis parmi le palmitate d'isopropyle, le myristate d'isopropyle, le stéarate d'octyldodécyle et l'isononanoate d'isononyle et leurs mélanges.

12. Composition selon l'une quelconque des revendications précédentes dans laquelle le ou les esters gras représentent de 0,01 % à 10 % en poids, de préférence de 0,1 % à 5 % en poids par rapport au poids total de la composition.

13. Composition selon l'une quelconque des revendications précédentes, **caractérisée par le fait que** les silicones sont choisies parmi les polyalkylsiloxanes, les polyarylsiloxanes, les polyalkylarylsiloxanes, les gommes et les résines de silicones, les polyorganosiloxanes modifiés par des groupements organofonctionnels ainsi que leurs mélanges.

14. Composition selon l'une quelconque des revendications précédentes, **caractérisée par le fait que** les silicones sont choisies parmi les polydialkylsiloxanes à groupements terminaux triméthylsilyle, les polydialkylsiloxanes à groupements terminaux diméthylsilanol et les polysiloxanes à groupements aminés.

15. Composition selon l'une quelconque des revendications précédentes dans laquelle le ou les silicones représentent de 0,01 % à 10 % en poids, de préférence de 0,3 % à 5 % en poids et plus particulièrement de 0,3 à 3 % en poids par rapport au poids total de la composition.

16. Procédé de traitement cosmétique non thérapeutique des matières kératiniques, notamment des fibres kératiniques, et en particulier des fibres kératiniques humaines telles que les cheveux, comprenant l'application sur lesdites matières d'une composition telle que décrite à l'une quelconque des revendications 1 à 15, la composition étant rincée ou non rincée après application.

17. Utilisation d'une composition telle que définie selon l'une des revendications 1 à 15 pour le soin et/ou la mise en forme des matières kératiniques, notamment des fibres kératiniques, et en particulier des fibres kératiniques humaines telles que les cheveux.
